# EUROPEAN PATENT APPLICATION

(11) **EP 2 305 680 A2**
(43) Date of publication of application: **06.04.2011**
(21) Application number: 10011796.9
(22) Date of filing: 29.09.2010
(51) Int. Cl.: C07D 473/16, A61K 31/52, A61P 31/14

(54) **Novel salts of (1S,4R)-cis-4-[2-amino-6-(cyclopropylamino)-9H-purin-9-yl]-2-cyclopentene-1-methanol**

(30) Priority: 30.09.2009 IN CH23852009
(71) Applicant: AUROBINDO PHARMA LIMITED, Hyderabad 500038 (IN)
(72) Inventor: Reddy, Narsihma Bobbali, 500 038, Andhra Pradesh (IN); Kaushik, Vipin Kumar, 500 038, Andhra Pradesh (IN); Islam, Aminul, 500 038, Andhra Pradesh (IN); Doddaveerappa, Anruth Gowda, 500 038, Andhra Pradesh (IN); Nagaprasad, Vishnubhotla, 500 038, Andhra Pradesh (IN); Sivakumaran, Meenakshisunderam, 500 038, Andhra Pradesh (IN)
(74) Representative: Wittkopp, Alexander

(57) **Abstract**

The present invention relates to novel salts of (1S,4R)-cis-4-[2-amino-6-(cyclopropylamino)-9H-purin-9-yl]-2-cyclopentene-1 -methanol of Formula I 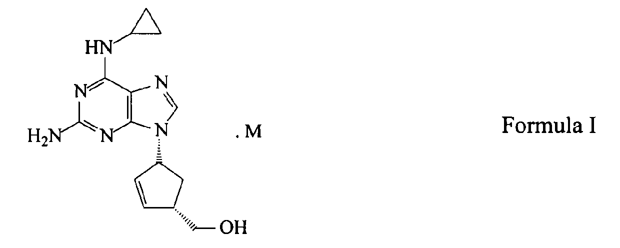 or solvates or hydrates thereof,
wherein M represents hemimalonate, malonate, hemioxalate, oxalate, hydrobromide, dihydrobromide, hemimaleate, maleate.

## Description

### FIELD OF THE INVENTION

The present invention relates to novel salts of (1S,4R)-cis-4-[2-amino-6-(cyclopropylamino)-9H-purin-9-yl]-2-cyclopentene-1-methanol of Formula I 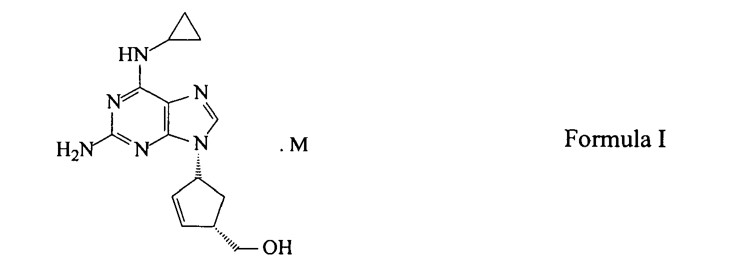 or solvates or hydrates thereof,
wherein M represents hemimalonate, malonate, hemioxalate, oxalate, hydrobromide, dihydrobromide, hemimaleate, maleate.

### BACKGROUND OF THE INVENTION

(IS,4R)-cis-4-[2-amino-6-(cyclopropylamino)-9H-purin-9-yl]-2-cyclopentene-1-methanol of Formula II 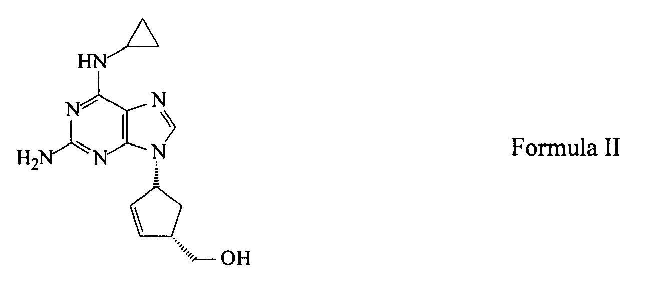 is generally known as Abacavir. Abacavir is an antiviral drug, especially effective against HIV infections.

US 5,034,394 discloses Abacavir and its pharmaceutically acceptable salts, base salts, e.g. derived from an appropriate base, such as alkali metal (e.g. sodium), alkaline earth metal (e.g. magnesium) salts, ammonium and NW⁺₄ (wherein W is C₁₋₄ alkyl). Physiologically acceptable salts of a hydrogen atom or an amino group include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids.

EP 0 777 669 B1 discloses succinate salt of Abacavir.

US 6,294,540 B1 discloses Abacavir hemisulfate, Abacavir glutarate, Abacavir hemisuberate, Abacavir adipate, Abacavir fumarate, Abacavir hemisebacate and Abacavir pimelate.

We have now found novel salts of Abacavir or solvates or hydrates thereof, which are stable and can be used in medical therapy particularly for the treatment of retroviral infections and hepatitis B viral infections.

### OBJECTIVE

The objective of the present invention is to provide novel salts of Abacavir or solvates or hydrates thereof.

Yet another objective of the present invention is to provide novel salts of Abacavir or solvates or hydrates thereof, which are crystalline in nature.

Yet another objective of the present invention is to provide novel salts of Abacavir or solvates or hydrates thereof, which are amorphous in nature.

### BRIEF DESCRIPTION OF THE DRAWINGS

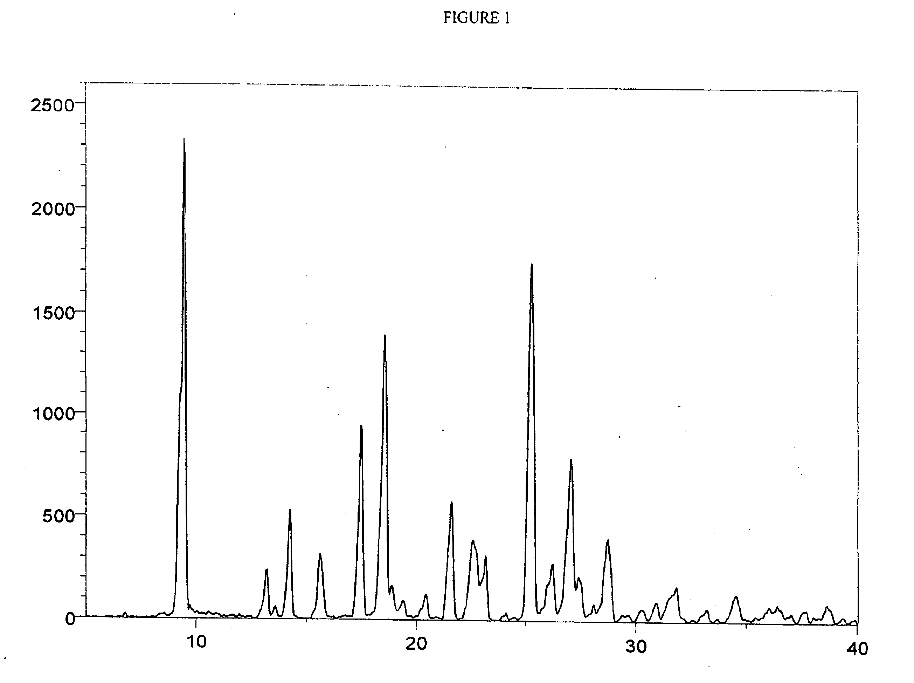

Figure 1 - Powder X-ray Diffraction (PXRD) of crystalline Abacavir malonate

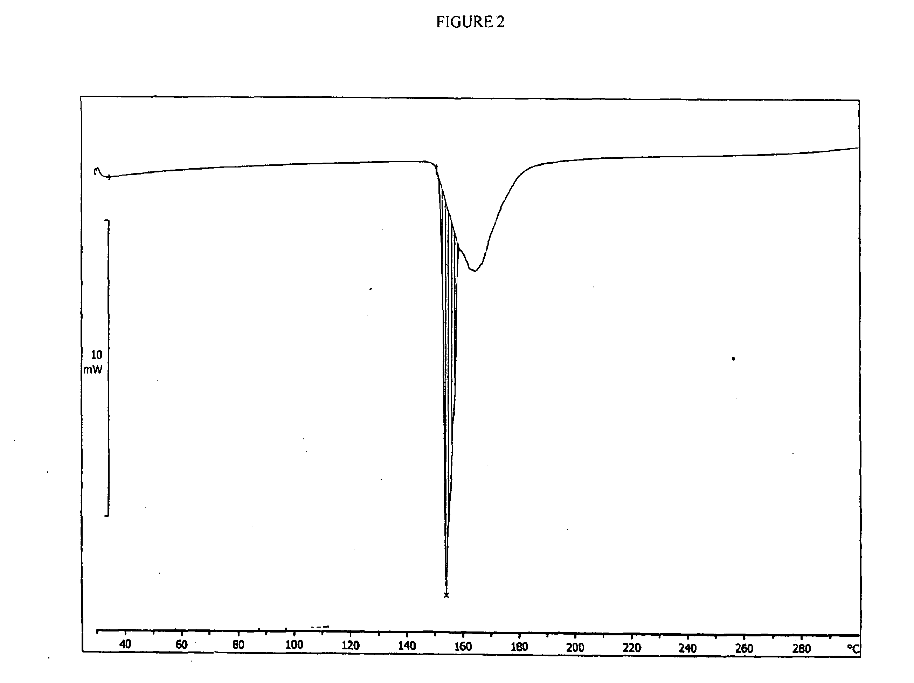

Figure 2 - Differential Scanning Calorimetry (DSC) of crystalline Abacavir malonate

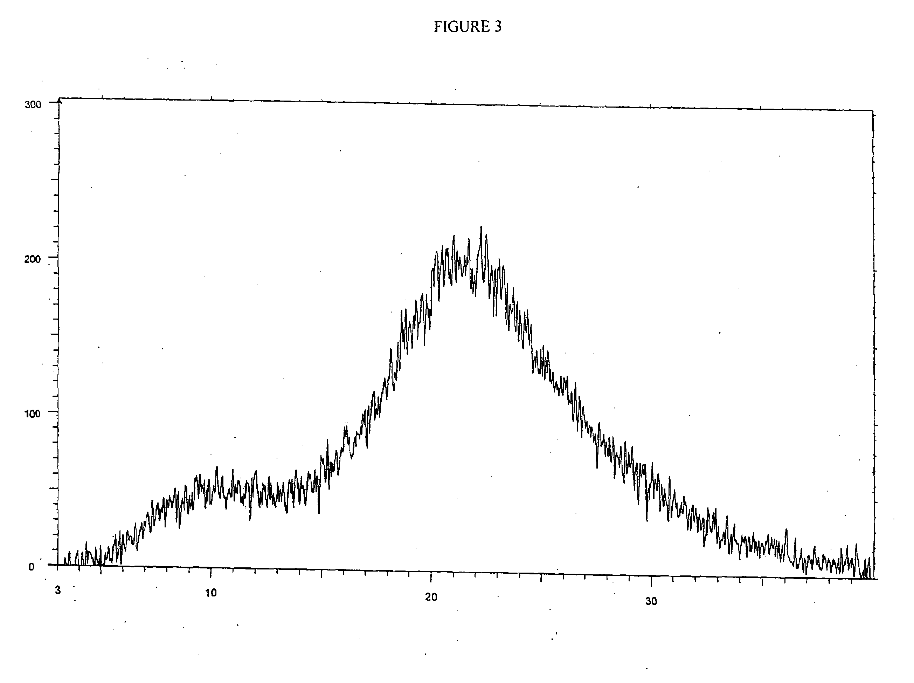

Figure 3 - Powder X-ray Diffraction (PXRD) of amorphous Abacavir malonate

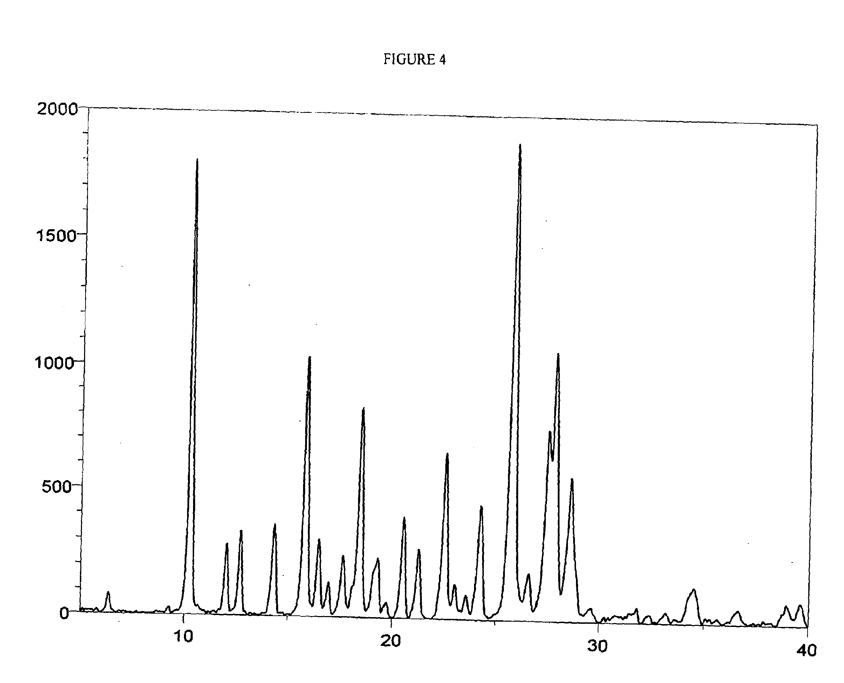

Figure 4 - Powder X-ray diffraction (PXRD) of crystalline Abacavir maleate

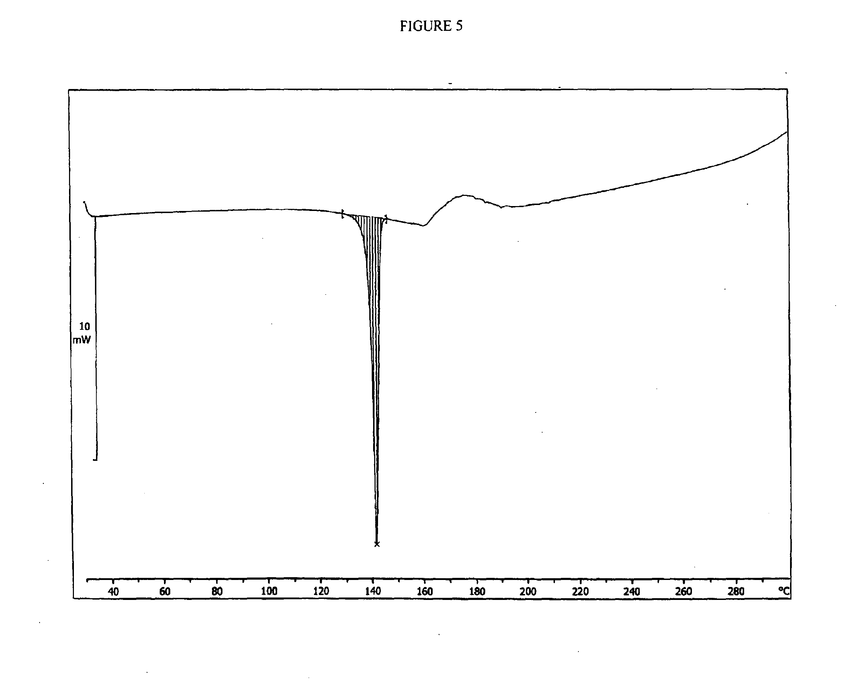

Figure 5 - Differential Scanning Calorimetry (DSC) of crystalline Abacavir maleate

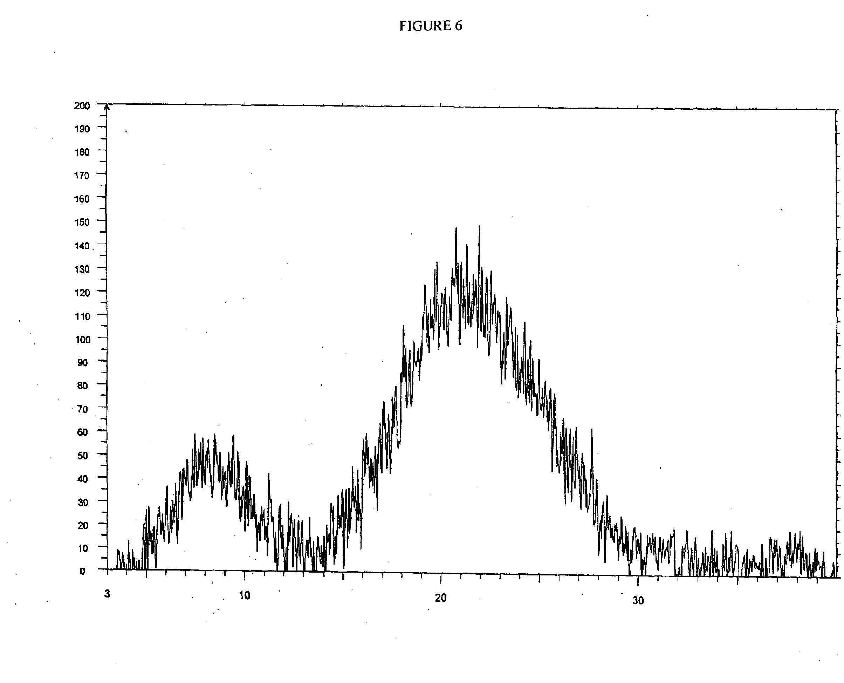

Figure 6 - Powder X-ray diffraction (PXRD) of amorphous Abacavir maleate

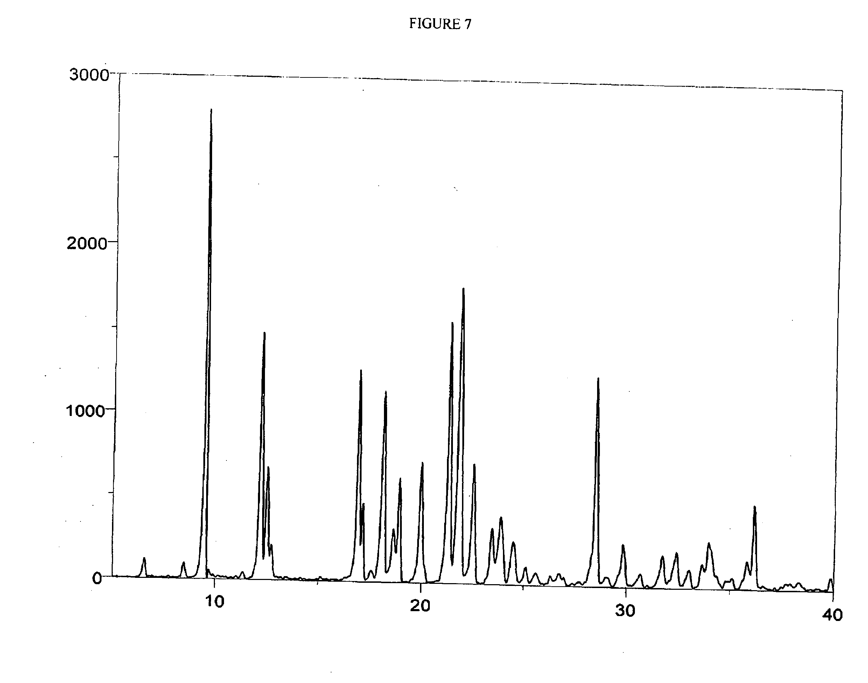

Figure 7 - Powder X-ray diffraction (PXRD) of crystalline Abacavir hemioxalate

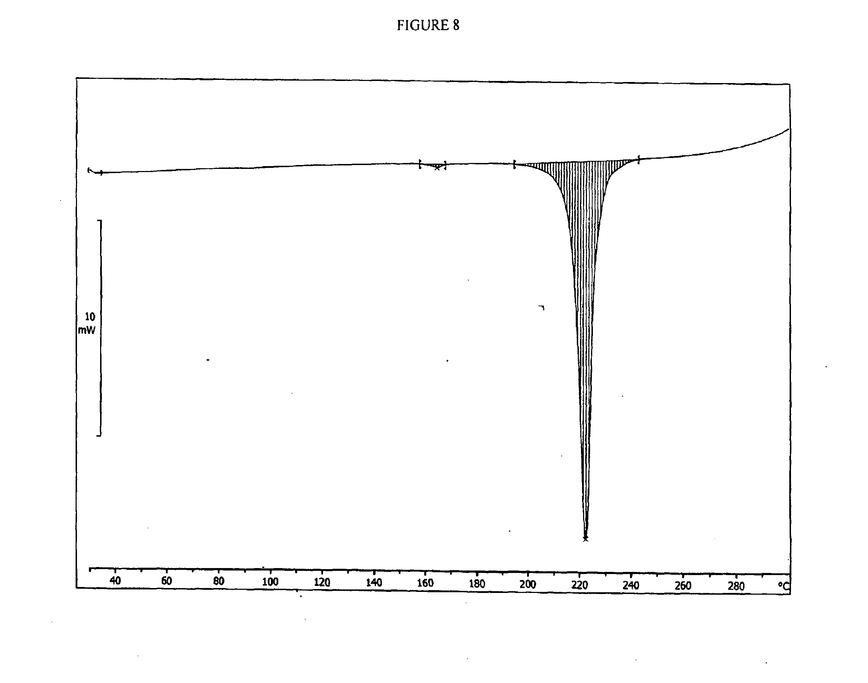

Figure 8 - Differential Scanning Calorimetry (DSC) of crystalline Abacavir hemioxalate

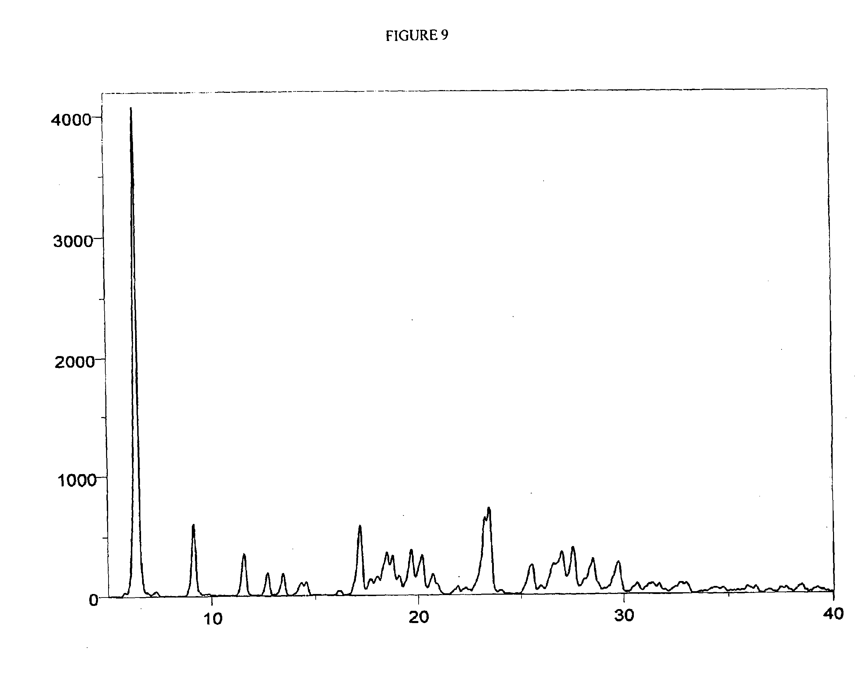

Figure 9 - Powder X-ray diffraction (PXRD) of crystalline Abacavir oxalate

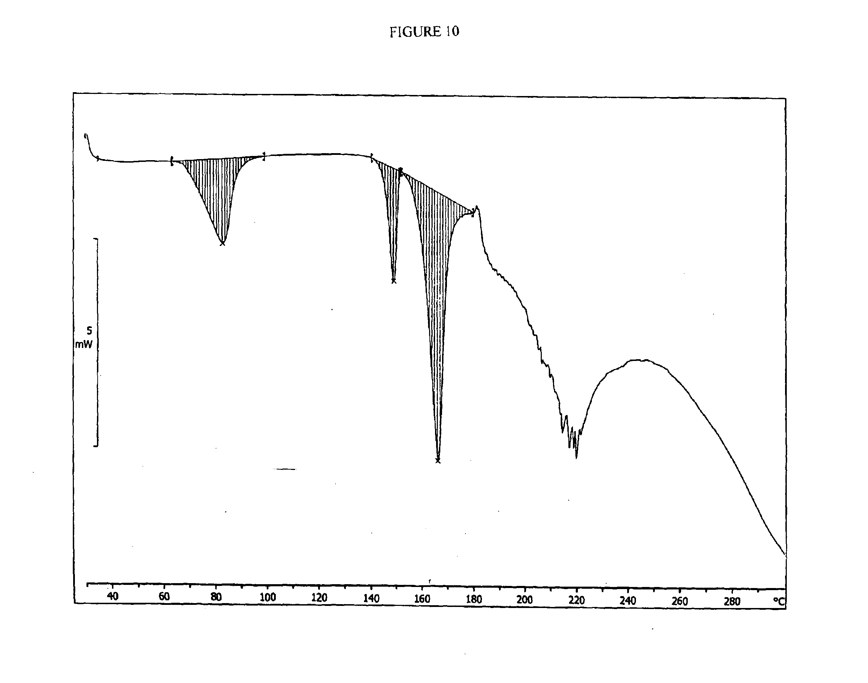

Figure 10 - Differential Scanning Calorimetry (DSC) of crystalline Abacavir oxalate

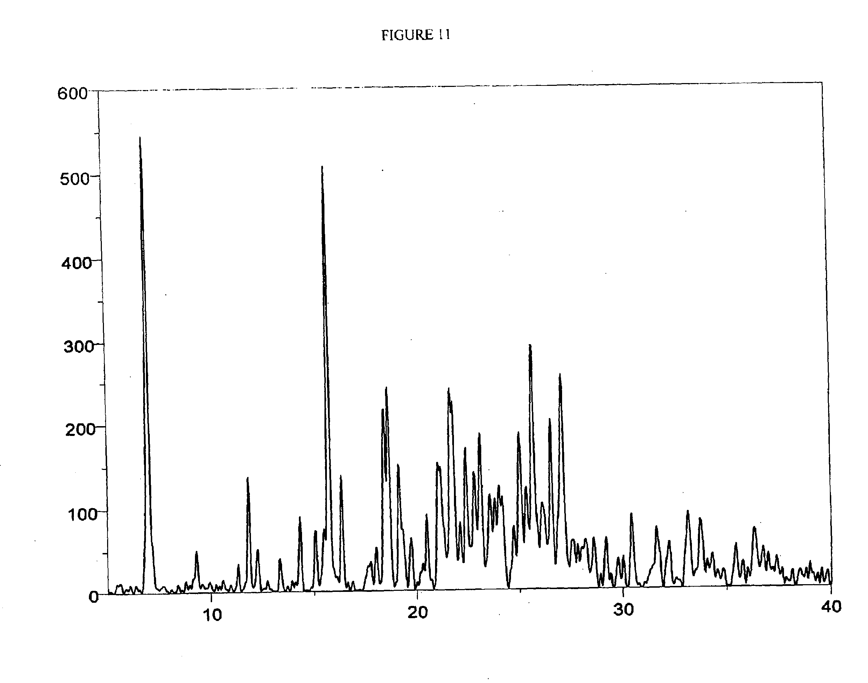

Figure 11 - Powder X-ray diffraction (PXRD) of crystalline Abacavir hydrobromide

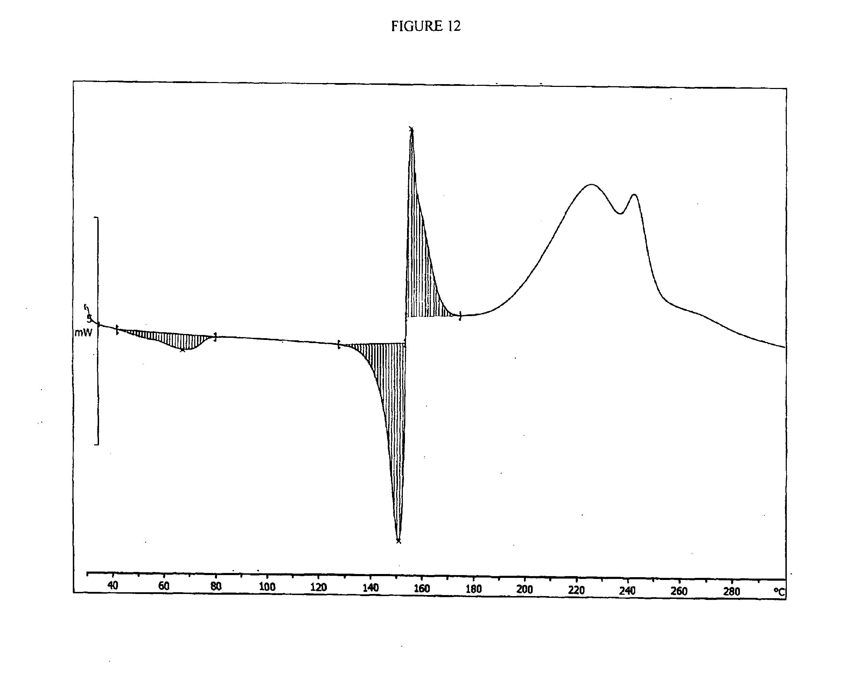

Figure 12 - Differential Scanning Calorimetry (DSC) of crystalline Abacavir hydrobromide

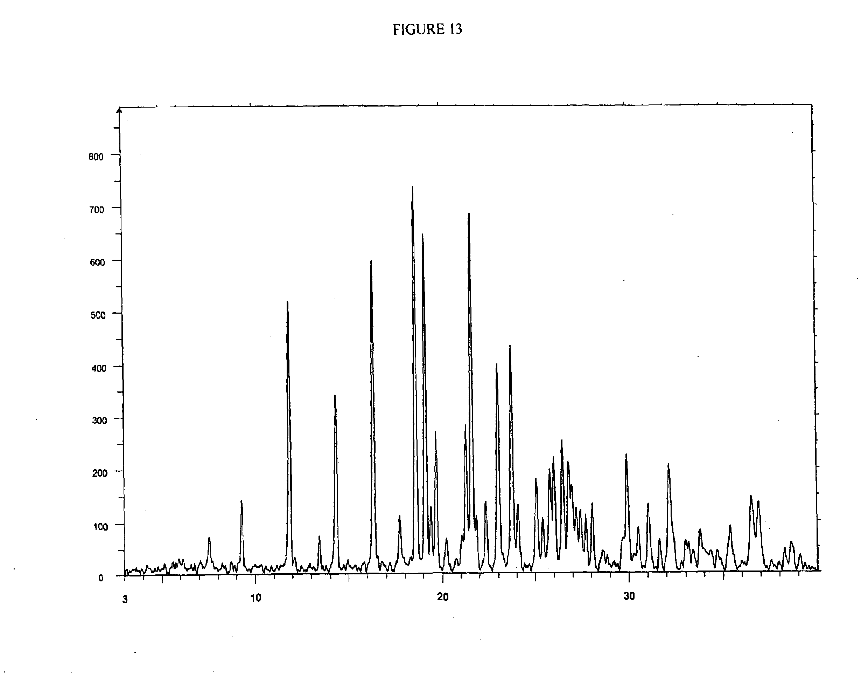

Figure 13 - Powder X-ray diffraction (PXRD) of crystalline Abacavir hydrobromide monohydrate

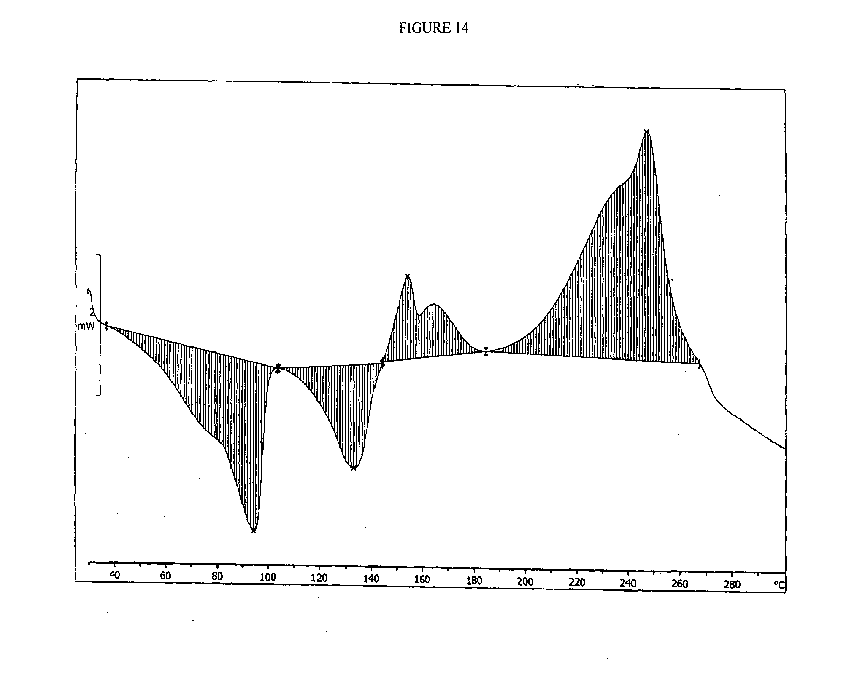

Figure 14 - Differential Scanning Calorimetry (DSC) of crystalline Abacavir hydrobromide monohydrate

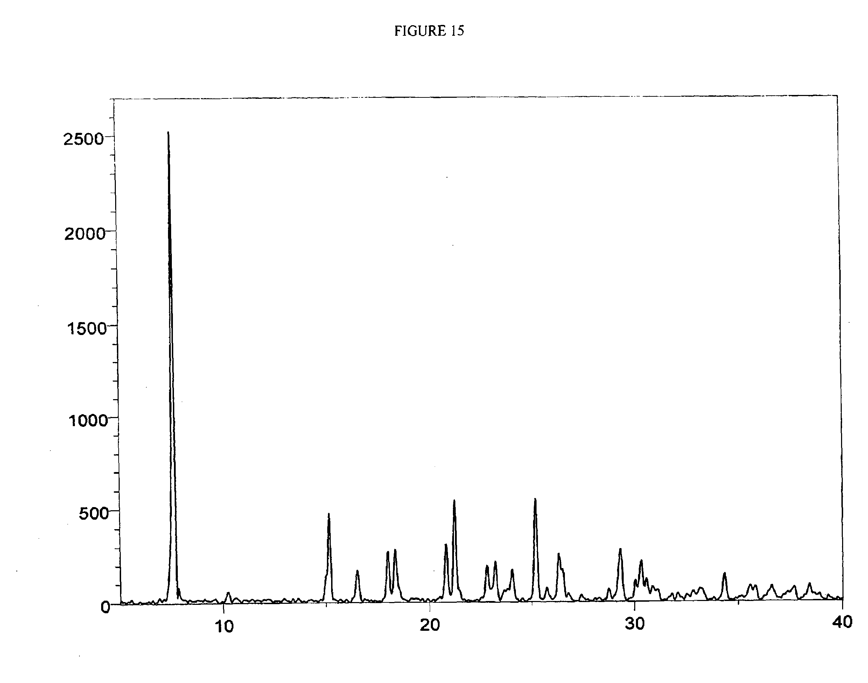

Figure 15 - Powder X-ray diffraction (PXRD) of crystalline Abacavir dihydrobromide

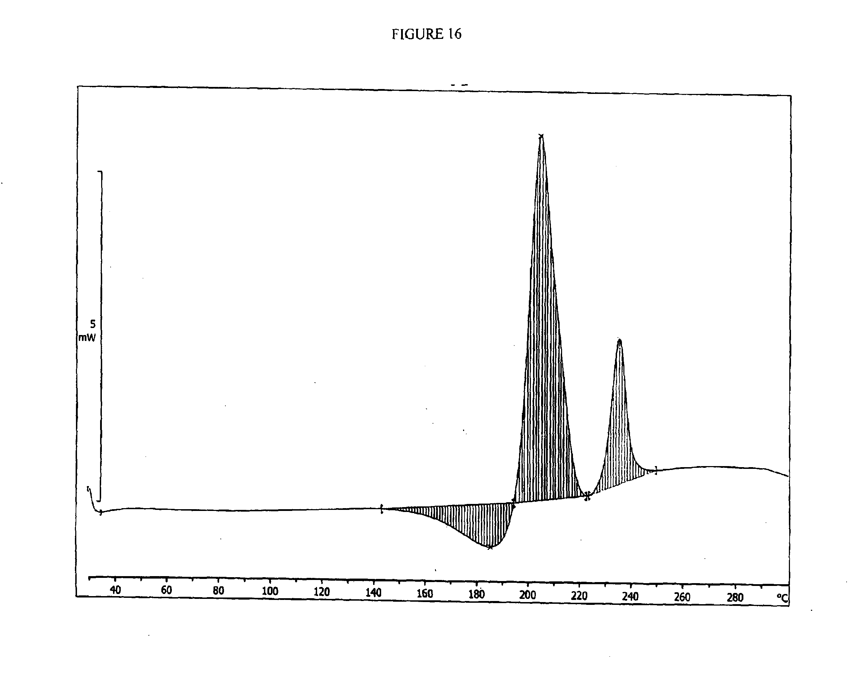

Figure 16 - Differential Scanning Calorimetry (DSC) of crystalline Abacavir dihydrobromide

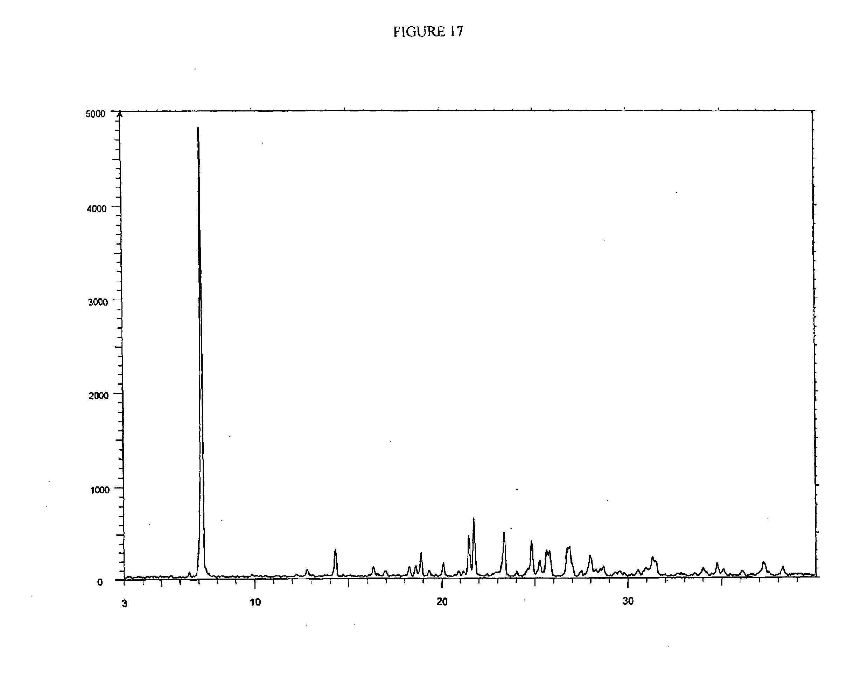

Figure 17 - Powder X-ray diffraction (PXRD) of crystalline Abacavir dihydrobromide monohydrate

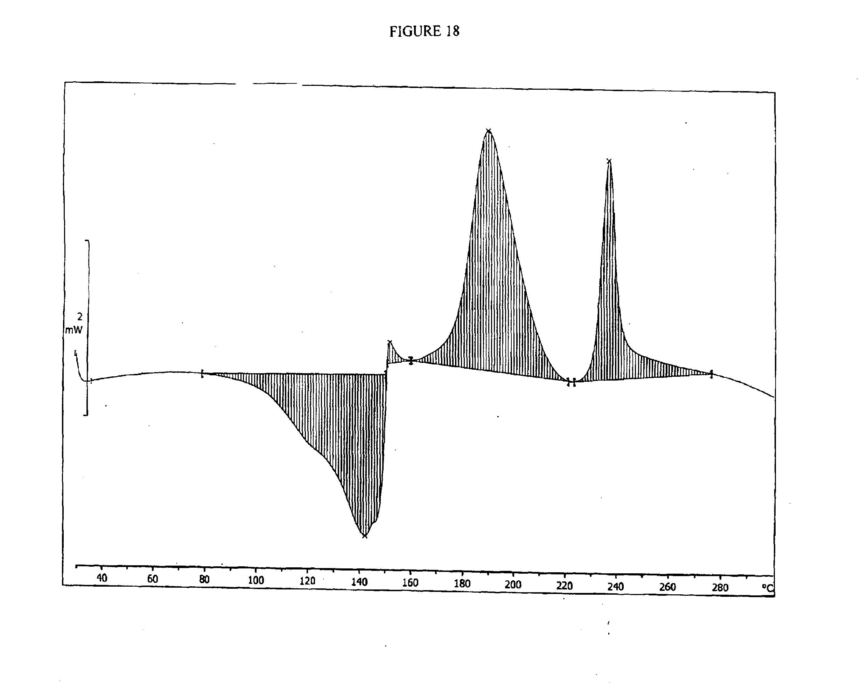

Figure 18 - Differential Scanning Calorimetry (DSC) of crystalline Abacavir dihydrobromide monohydrate

### SUMMARY OF THE INVENTION

The present invention relates to novel salts of (1S,4R)-cis-4-[2-amino-6-(cydopropylamino)-9H-purin-9-yl]-2-cyclopentene-1-methanol of Formula I 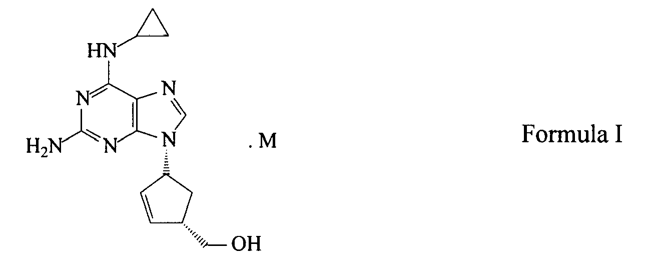 or solvates or hydrates thereof,
wherein M represents hemimalonate, malonate, hemioxalate, oxalate, hydrobromide, dihydrobromide, hemimaleate, maleate.

In another embodiment the present invention also relates to the novel salts of (1S,4R)-cis-4-[2-amino-6-(cyclopropylamino)-9H-purin-9-yl]-2-cyclopentene-1-methanol or solvates or hydrates thereof, which are crystalline.

In another embodiment the present invention also relates to the novel salts of (1S,4R)-cis-4-[2-amino-6-(cyclopropylamino)-9H-purin-9-yl]-2-cyclopentene-1-methanol or solvates or hydrates thereof, which are amorphous.

In another embodiment the present invention also relates to the process for preparing novel salts of (1S,4R)-cis-4-[2-amino-6-(cydopropylamino)-9H-purin-9-yl]-2-cyclopentene-1-methanol or solvates or hydrates thereof.

### DETAILED DESCRIPTION OF THE INVENTION

The present invention relates to novel salts of Abacavir of Formula I 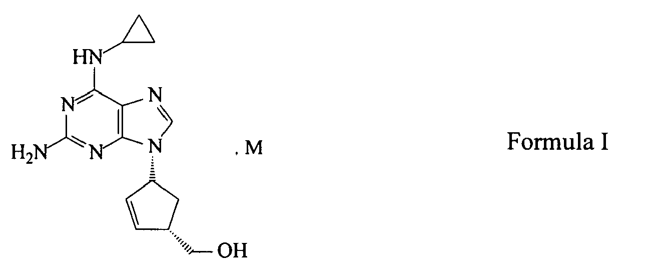 or solvates or hydrates thereof,
wherein M represents hemimalonate, malonate, hemioxalate, oxalate, hydrobromide, dihydrobromide, hemimaleate, maleate.

Another aspect of the present invention relates to novel salt of Abacavir malonate of Formula III 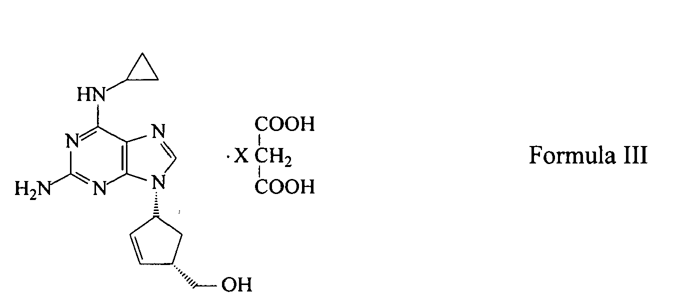 or solvates or hydrates thereof.
wherein X represents ½ or 1

In an aspect of the present invention, crystalline Abacavir malonate having Powder X-ray diffraction °2θ values at 9.47, 13.2, 14.2, 15.6, 17.5, 18.5, 21.6, 22.5, 23.1, 25.2, 26.1, 27.0, 28.6 ±0.2.

In an aspect of the present invention, crystalline Abacavir malonate has Differencial scanning calorimetry (DSC) endothermic peak at 153.3°C.

Yet another aspect of the present invention relates to a process to prepare crystalline form of Abacavir malonate salt, comprising:
i) treating Abacavir with malonic acid in a solvent;
ii) isolating Abacavir malonate salt,
wherein Abacavir is a free base or a salt other than malonate; salt is isolated as hemi-malonoate or malonoate; the solvent is selected from polar solvents (such as water, methanol, ethanol, isopropyl alcohol, butanol, ethylacetate, methylacetate, acetonitrile or acetone), non-polar solvents (such as methyl t-butyl ether, tetrahydrofuran, diisopropyl ether, toluene, hexanes or heptanes) or mixtures thereof.

Another aspect of the present invention relates to Abacavir malonate in amorphous form.

Yet another aspect of the present invention relates to a process to prepare amorphous form of Abacavir malonate salt, comprising:
i) treating Abacavir with malonic acid in a solvent;
ii) removing the solvent; and
iii) isolating the Abacavir malonate salt,
wherein Abacavir is a free base or a salt other than malonate; salt is isolated as hemi-malonoate or malonoate; the solvent is selected from polar solvents, non-polar solvents or mixtures thereof.

The removal of solvent is carried out using distillation, spray-drying, freeze-drying or lyophilization.

Another aspect of the present invention relates to novel salt of Abacavir oxalate of Formula IV 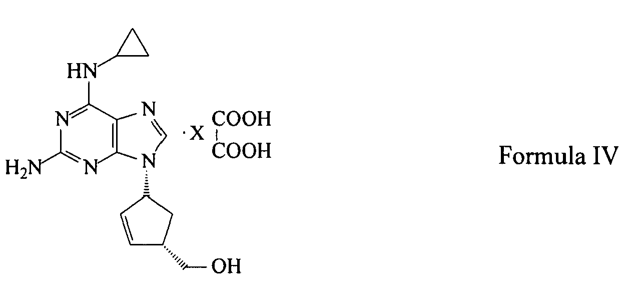 or solvates or hydrates thereof,
wherein X represents ½ or 1.

In an aspect of the present invention, crystalline Abacavir hemioxalate having Powder X-ray diffraction °2θ values at 9.4, 12.1, 12.5, 16.9, 17.1, 18.1, 18.6, 18.9, 19.9, 21.3, 21.8, 22.5, 23.4, 23.8, 28.4, 33.9, 36.1 ±0.2.

In an aspect of the present invention, crystalline Abacavir hemioxalate has DSC endothermic peaks at 164.3°C and 221.4°C.

In an aspect of the present invention, Abacavir oxalate in crystalline form, having Powder X-ray diffraction °2θ values at 6.4, 9.1, 17.1, 23.4, 27.5 ±0.2.

In an aspect of the present invention, Abacavir oxalate in crystalline form, having DSC endothermic peaks at 82.5°C, 148.6°C and 165.6°C.

Yet another aspect of the present invention relates to a process to prepare crystalline form of Abacavir oxalate, comprising:
i) treating Abacavir with oxalic acid in a solvent;
ii) isolating Abacavir oxalate salt,
wherein Abacavir is free base or a salt other than oxalate; salt is isolated as hemi-oxalate or oxalate salt; the solvent is selected from polar solvents (such as water, methanol, ethanol, isopropyl alcohol, butanol, ethylacetate, methylacetate, acetonitrile or acetone), non-polar solvents (such as methyl t-butyl ether, tetrahydrofuran, diisopropyl ether, toluene, hexanes or heptanes) or mixtures thereof.

Another aspect of the present invention relates to novel salt of Abacavir maleate of Formula V, 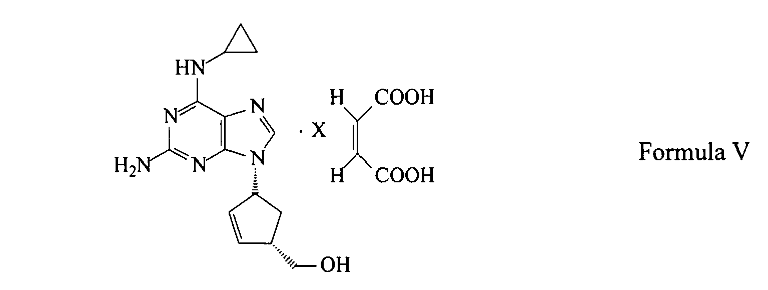 or solvates or hydrates thereof,
wherein X represents ½ or 1.

In an aspect of the present invention, crystalline Abacavir maleate having Powder X-ray diffraction °2θ values at 10.2, 12.0, 12.6, 14.3, 15.8, 16.4, 17.6, 18.4, 19.2, 20.5, 21.2, 22.5, 23.0, 24.2, 25.7, 26.5, 27.4, 27.7, 28.5 ±0.2.

In an aspect of the present invention, crystalline Abacavir maleate has DSC endothermic peak at 141.0°C.

Yet another aspect of the present invention relates to a process to prepare crystalline form of Abacavir maleate, comprising:
i) treating Abacavir with maleic acid in a solvent;
ii) isolating Abacavir maleate salt,
wherein Abacavir is a free base or a salt other than maleate; salt is isolated as hemimaleate or maleate; the solvent is selected from polar solvents (such as water, methanol, ethanol, isopropyl alcohol, butanol, ethylacetate, methylacetate, acetonitrile or acetone), non-polar solvents (such as methyl t-butyl ether, tetrahydrofuran, diisopropyl ether, toluene, hexanes or heptanes) or mixtures thereof.

In an aspect of the present invention Abacavir maleate is in amorphous form.

Yet another aspect of the present invention relates to a process to prepare amorphous form of Abacavir maleate salt, comprising:
i) treating Abacavir with maleic acid in a solvent;
ii) removing the solvent; and
iii) isolating Abacavir maleate salt,
wherein Abacavir is a free base or a salt other than maleate; salt is isolated as hemimaleate or maleate; the solvent is selected from polar solvents (such as water, methanol, ethanol, isopropyl alcohol, butanol, ethylacetate, methylacetate, acetonitrile or acetone), non-polar solvents (such as methyl t-butyl ether, tetrahydrofuran, diisopropyl ether, toluene, hexanes or heptanes) or mixtures thereof.

The removal of solvent can be carried out using distillation, spray-drying, freeze-drying or lyophilization.

Another aspect of the present invention relates to novel salt of Abacavir hydrobromide of Formula VI, 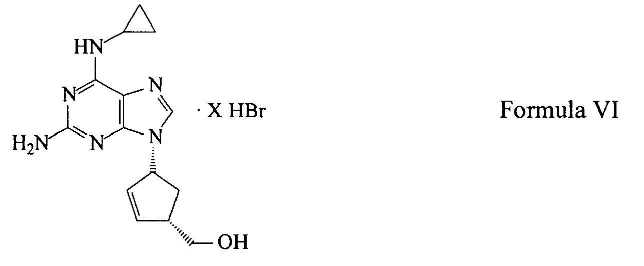 or solvates or hydrates thereof,
wherein X represents 1 or 2.

In an aspect of the present invention, crystalline Abacavir hydrobromide having Powder X-ray diffraction °2θ values at 6.9, 11.8, 15.7, 16.3, 18.4, 18.6, 19.1, 21.0, 21.6, 22.4, 22.8, 23.1, 23.5, 23.9, 25.0, 25.6, 26.5, 27.1 ±0.2.

In an aspect of the present invention, crystalline Abacavir hydrobromide has DSC endothermic peaks at 67.2°C, 150.5°C and exothermic peak at 155.8°C.

In an aspect of the present invention, crystalline Abacavir di-hydrobromide having Powder X-ray diffraction °2θ values at 7.6, 15.1, 18.0, 18.3, 20.8, 21.2, 25.1, 26.3, 29.3 ±0.2.

In an aspect of the present invention, crystalline Abacavir di-hydrobromide has DSC endothermic peak at 185.2°C and exothermic peaks at 204.6°C and 235.2°C.

Yet another aspect of the present invention relates to a process to prepare crystalline form of Abacavir hydrobromide, comprising:
i) treating Abacavir with hydrobromic acid in a solvent;
ii) isolating Abacavir hydrobromide salt,
wherein Abacavir is a free base or a salt other than hydrobromide; salt is isolated as hydrobromide or dihydrobromide salt; the solvent is selected from polar solvents (such as water, methanol, ethanol, isopropyl alcohol, butanol, ethylacetate, methylacetate, acetonitrile or acetone), non-polar solvents (such as methyl t-butyl ether, tetrahydrofuran, diisopropyl ether, toluene, hexanes or heptanes) or mixtures thereof.

In an aspect of the present invention, crystalline Abacavir hydrobromide monohydrate having Powder X-ray diffraction °2θ values at 7.5, 9.2, 11.8, 13.4, 14.3, 16.3, 17.7, 18.6, 19.1, 19.6, 21.3, 21.6, 22.3, 23.0, 23.7, 24.1, 25.1, 25.8, 26.0, 26.5, 26.8, 28.0, 29.9, 30.5, 31.1, 31.6, 32.4, 36.5, 36.9 ±0.2.

In an aspect of the present invention, crystalline Abacavir hydrobromide monohydrate has DSC endothermic peaks at 94.1 °C, 133.0°C and exothermic peak at 153.8°C, 247.1 °C.

In an aspect of the present invention, crystalline Abacavir dihydrobromide monohydrate having Powder X-ray diffraction °2θ values at 7.1, 14.2, 18.8, 21.4, 21.7, 23.3, 24.8, 25.7, 26.8, 27.9, 31.3 ±0.2.

In an aspect of the present invention, crystalline Abacavir dihydrobromide monohydrate has DSC endothermic peaks at 142.0°C and exothermic peak at 151.6°C, 190.1°C, 237.1°C.

Another embodiment of the present invention, the novel salts of the present invention are stable and non-hygroscopic.

The polar solvent is selected from the group consisting of water, methanol, ethanol, isopropyl alcohol, butanol, ethylacetate, methylacetate, acetonitrile, acetone.

The non-polar solvent is selected from the group consisting of methyl t-butyl ether, tetrahydrofuran, diisopropyl ether, toluene, hexanes, heptanes.

Abacavir is prepared by using processes reported in literature.

In another embodiment of the invention there are provided the compounds according to the invention for use in medical therapy particularly for the treatment of retroviral infections and hepatitis B viral infections. Examples of retroviral infections which may be treated or prevented in accordance with the invention include human retroviral infections such as human immunodeficiency virus (HIV), HIV-1, HIV-2 and human T-cell lymphotropic virus (HLTV), e.g. HTLV-1 or HTLV-II infections. The compounds according to the invention are especially useful for the treatment of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions and thrombocytopenic purpura. The compounds may also be used in the treatment or prevention of psoriasis.

The compounds of the invention may be administered alone or in combination with other therapeutic agents suitable in the treatment of HIV infections, such as Nucleoside Reverse Transciptase Inhibitors (NRTIs) for example zidovudine, zalcitabine, lamivudine, didanosine, stavudine, 5-chloro-2',3'-dideoxy-3'-fluorouridine, adefovir and (2R,5S)-5-fluoro-1-[2-(hydroxymethyl)-1,3-oxathiolan-5-yl]cytosine, lovaride; non-NRTIs for example nevirapine, delavuridine, α-APA, HBY-1293 and efavirenz; HIV protease inhibitors for example saquinavir, indinavir, nelfinavir, ritonavir and VX-478, immune modulators for example interleukin II, erythyropoetin, tucaresol, and interferons for example α-interferon. In addition the compound of the invention may be administered in combination with other therapeutic agents suitable in the treatment of HBV infections for example lamivudine, emtricitabine, immune modulators, and interferons. Such combinations may be administered together or sequentially providing that any duration between the administration of each therapeutic agent does not diminish their additive effect.

In view of their useful pharmacological properties, the compounds according to the present invention may be formulated into various pharmaceutical forms for administration purposes. While it is possible for the compound of the present invention to be administered as the raw chemical, it is preferable and advantageous to present the compound of the invention as a pharmaceutical formulation, and represents a further embodiment of the invention.

The pharmaceutical formulation comprises the compounds of the invention as active ingredient optionally together with one or more pharmaceutically acceptable carrier(s) therefore and optionally other therapeutic agents. The carrier(s) must be "pharmaceutically acceptable" in the sense of being compatible with the other ingredients and the active ingredient of the formulation and not deleterious to the recipients thereof.

The compounds according to the invention may be administered by any route appropriate to the condition to be treated, suitable routes including, but not limited to, oral, rectal, nasal, topical (including, but not limited to, transdermal, buccal and sublingual), vaginal and parenteral (including, but not limited to, subcutaneous, intramuscular, intravenous, intradermal, intraarticular, intrathecal and epidural). It will be appreciated that the preferred route may vary with, for example, the condition of the recipient.

For each of the above-indicated utilities and indications the amount required of the individual active ingredient will depend upon a number of factors including the severity of the condition to be treated and the identity of the recipient and will ultimately be at the discretion of the attendant physician. In general, however, for each of these utilities and indications, a suitable, effective dose will be in the range of 1 to 1000 mg per day. The dose may if desired be presented as two, three, four or more sub-doses administered at appropriate intervals throughout the day.

The formulations can be presented in any dosage form, including unit dosage form and may be prepared by any of the methods well known in the art. To prepare the pharmaceutical compositions of this invention, an effective amount of the compound of the present invention, as the active ingredient is optionally combined with a pharmaceutically acceptable carrier, which may take a wide variety of forms depending on the form of preparation desired for administration.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an emulsion. The active ingredient may also be presented as a bolus or paste or may be contained within liposomes.

A tablet formulation can be prepared by any process known in the art, including wet granulation, dry granulation/compaction or direct compression or moulding. Compressed tablets may be prepared by compressing in a suitable machine the pharmaceutical composition comprising active ingredient optionally with one or more pharmaceutically acceptable carrier. Examples of pharmaceutically acceptable carrier(s) include diluents, binders, disintegrant, lubricant, glidant, surface-active or dispersing agent. Moulded tablets may be made by moulding in a suitable machine a mixture of the powdered compound moistened with an inert liquid diluent. The tablets may optionally be coated or scored and may be formulated so as to provide modified or controlled release of the active ingredient therein, using various techniques for controlled release or modified release well known in the art.

A capsule may be made by filling a loose or compressed powder, pellets, granules, mini-tablets, tablets, microsphere or the like or combinations thereof, optionally with one or more pharmaceutically acceptable carriers using appropriate capsule filling machine. Examples of pharmaceutically acceptable carriers include binders, glidants, lubricants, diluents, disintegrants, and surface active or dispersing agents. Capsules may also be formulated to provide slow or controlled release of the active ingredient. This can be achieved by using various techniques for controlled release or modified release well known in the art.

For buccal administration the formulation may take the form of tablets or lozenges formulated in conventional manner.

Diluents, also termed "fillers," are typically necessary to increase the bulk of a solid dosage form so that a practical size is provided for compression of tablets or formation of beads and granules. Suitable diluents include, but are not limited to, dicalcium phosphate, calcium sulfate, lactose, sucrose, mannitol, sorbitol, cellulose, cellulose derivatives, microcrystalline cellulose, kaolin, sodium chloride, dry starch, hydrolyzed starches, starch derivatives, pre-gelatinized starch, silicone dioxide, titanium oxide, magnesium aluminum silicate and sugars. The amount of diluents in the formulation can range from about 20 to about 90% by weight of the total formulation.

Binders refers to substances that has binding properties, thus serve as the "adhesive" in the formulation. Binders according to the present invention can be selected from the group comprising, but not limited to, water-soluble polymer, water insoluble polymer, gums such as acacia, gelatin and tragacanth; derivatives of seaweed such as alginic acid, sodium alginate and ammonium calcium alginate; waxes, gelling agents, polysaccharides or the like. Preferably can be water soluble polymers for example hydroxypropyl methyl cellulose, hydroxypropyl cellulose, hydroxyethyl cellulose, povidone, starch and its derivatives, cellulose derivatives, methyl cellulose or the like. The amount of binder in the formulation can range from, but not limited to, about 1 to about 10% by weight of the total formulation.

Glidants refers to material that prevents caking and improves the flow characteristics of granulations, so that flow is smooth and uniform. Suitable glidants include colloidal silicon dioxide and talc. The amount of glidant in the formulation can range from about 0.01 % to about 5% by weight of the total formulation.

Lubricants are substances added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; sodium stearyl fumarate; high melting point waxes such as glyceryl behenate; and water soluble lubricants such as boric acid sodium chloride, sodium benzoate, sodium acetate, sodium chloride sodium oleate, polyethylene glycols and d'1-leucine. The amount of lubricant in the formulation can range from about 0.1 to about 10% by weight of the tablet formulation.

Disintegrant refers to materials added to the formulation to help it break apart (disintegrate) and release the drug. Suitable disintegrants include starches; modified starches such as sodium starch glycolate; natural and synthetic gums such as locust bean, karaya, guar gum, tragacanth and agar; cellulose derivatives such as methylcellulose, low-substituted hydroxypropyl cellulose and sodium carboxymethylcellulose; microcrystalline celluloses and cross-linked polymers such as sodium croscarmellose, crospovidone; alginates such as alginic acid and sodium alginate; clays such as bentonites; and effervescent mixtures. The amount of disintegrant in the composition can range from about 2 to about 15% by weight of the formulation.

Formulations of the present invention suitable for parenteral administration may be presented in the form of parenteral solutions, suspension, liposomes, nanosomes, emulsions or the like. The parenteral solutions may be prepared by dissolving active ingredient in pharmaceutically acceptable carriers/solvents including aqueous or non-aqueous carriers optionally along with other ingredients including but not limited to, buffering agent, isotonicity agent, suspending agent, preservatives, stabilizing agent, viscosity modifier or combinations thereof. Injectable solutions, for example, may be prepared in which the carrier comprises sterile water, saline solution, glucose solution or a mixture of saline and glucose solution. Injectable solutions containing non-aqueous carrier may be formulated in oil for prolonged action.

Solubility is the property of a solid, liquid, or gaseous chemical substance called *solute* to dissolve in a liquid solvent to form a homogeneous solution of the solute in the solvent. The extent of the solubility of a substance in a specific solvent is measured as the saturation concentration where adding more solute does not increase the concentration of the solution.

The saturation solubility of the compounds according to the present invention is measured in various solvent systems including simulated gastric fluid, pH 4.5 buffer, pH 6.8 buffer solution and pH 7.2 buffer solution. The compounds according to the present invention show better solubility as compared to abacavir sulphate in all solvent systems.

**Table- 1: Comparison of saturation solubility of Abacavir malonate with Abacavir sulphate (marketed salt)**

| **S. No** | **Solvent** | **Solubility (mg/ml)** | |
|---|---|---|---|
| | | **Abacavir Sulphate** | **Abacavir Malonate** |
| 1 | Water | 89.2 | 116.2 |
| 2 | O.1N HC1 | 93.1 | 368.4 |
| 3 | O.O1N HC1 | - | 733.3 |
| 4 | pH 4.5 Sodium acetate buffer | 112.6 | 403.6 |
| 5 | pH 6.8 Phosphate buffer | 89.5 | 425.2 |
| 6 | pH 7.2 Phosphate buffer | 124.0 | 453.5 |

### POWDER X-RAY DIFFRACTION (PXRD)

The X-ray powder diffractogram is obtained using a Seifert, XRD 3003 TT system. The X-ray generator was operated at 40 kv and 30 mA, using the Kαl radiation source. It is scanned in the diffraction range of 3° to 40° 2θ at a scan rate of 0.03° 20 per second.

### DIFFERENCIAL SCANNING CALORIMETRY (DSC)

The thermal behavior of Abacavir salts is examined by DSC, using Mettler Toledo - instrument Model DSC821^{e}, caliberated with Indium and Zinc. The sample is heated in a pierced aluminium pan under nitrogen atmosphere at a rate of 10°C/min over a temperature range of 30°C to 200°C.

The invention is illustrated with the following examples, which are provided by way of illustration only and should not be construed to limit the scope of the invention.

### EXAMPLE-1

### PREPARATION OF (1S,4R) 4-[2-AMINO-6-(CYCLOPROPYLAMINO)-9H-PURIN-9-YL]-2-CYCLOPENTENE-1-METHANOL (ABACAVIR BASE)

Cyclopropylamine (147.20 g, 2.582 mol) was added to a suspension of (1S,4R)-4-(2-amino-6-chloro-9H-purin-9-yl)-2-cyclopentene-1-methanolhydrochloride (150 g, 0.497 mol) in ethanol (absolute alcohol, 1500 ml) at 25-30°C and reaction mass was heated to reflux at 74-78°C for -6 hrs to complete the reaction. Thereafter, the reaction mass was concentrated at 40-50°C under reduced pressure (700-50 mm of Hg) to remove the solvents completely. Obtained concentrated mass was diluted with DM water (450 ml) & ethyl acetate (1500 ml) at 25-45°C and pH was adjusted to 40-45°C. Organic layer was separated and aqueous layer, saturated with sodium chloride (150g) was re-extracted with preheated ethyl acetate (1 x 900 ml, 1 x 450 ml, 40-45°C). Combined organic layer was treated with carbon enoanticromos and concentrated at 40-50°C under reduced pressure (200-50 mm of Hg) to yield a foamy solid i.e. (1S,4R)-4-[2-amino-6-(cyclopropylamino)-9H-purin-9-yl]-2-cyclopentene-1-methanol (Abacavir base).

### EXAMPLE-2

### PREPARATION OF (1S,4R)-4-[2-AMINO-(6-CYCLOPROPYLAMINO)-9H-PURIN-9- YL]-2-CYCLOPENTENE-1-METHANOL (ABACAVIR BASE)

Abacavir hemisulfate (300 g, 0.453 mol) was suspended in mixture of ethyl acetate (3000 ml) and DM water (900 ml) at 25-30°C. The contents were heated to 40-45°C and pH of the suspension was adjusted to 6.0-6.1 with 20% w/w aqueous sodium hydroxide solution (-250 ml). Thereafter, organic layer was separated and aqueous layer was re-extracted with ethyl acetate (1 x 600 ml) at 40-45°C. Carbon enoanticromos (15 g) was added to the combined organic layer and contents were stirred for ∼1 h at 40-45°C. Carbon was removed through hyflo and residue was washed with pre-heated ethyl acetate (2 x 100 m1,40-45°C), Concentrated the filtrate at 50-60°C under reduced pressure (150-50 mm Hg) to obtain a foamy oily mass. Thereafter, acetone (1000 ml) was added to the concentrated mass and stirred for ∼30 min at 0-5°C. Product was filtered, washed with acetone (2 x 100 ml) under nitrogen atmosphere and dried at 40-50°C under reduced pressure (10-50 mm Hg) to a constant weight.
**Yield:** 200 g (78% of Theory)
**Chromatographic Purity (by HPLC)** : 99.86%
**Chiral Purity (by HPLC)** :99.93%
**Assay (by HPLC, % w/w)** : 99.4

### EXAMPLE-3

### PREPARATION OF (1S,4R)-4-[2-AMINO-6-CHLORO-9H-PURIN-9-YL]-2-CYCLOPENTENE-1-METHANOL (CHLOROPURINE BASE)

(1S,4R)-4-(2-Amino-6-chloro-9H-purin-9-yl)-2-cyclopentene-1-methanol hydrochloride (50 g, 0.1656 moles) was suspended in DM water (400 ml, 25-30°C) and contents were further cooled to 10-15°C. Thereafter, pH of reaction mixture was adjusted to 7.0-7.5 with ∼10% w/w aqueous ammonia solution (25 ml) slowly in a period of ∼60 min at 10-15°C. Product was filtered, washed with pre-cooled DM water (100 ml) and dried at 40-50°C/∼10mmHg to at constant weight.
Yield: 38 g
Chromatographic Purity (by HPLC): 98.55%

### EXAMPLE-4

### PREPARATION OF CRYSTALLINE FORM OF ABACAVIR MALONATE

Malonic acid (14.2 g, 0.136 mole) dissolved in 150 ml of ethanol was added to a suspension of Abacavir base (30 g, 0.105 mole) in 300 ml of ethanol in a period of ∼15 min at 25-30°C. The contents were heated to 60-70°C and stirred for -30 min to obtain a clear solution. Thereafter, obtained solution was cooled to 40-45°C. Precipitated product was further cooled to 5-10°C and stirred for -2 h. Product was filtered, washed with pre-cooled ethanol (1 x 30 ml, 5-10°C) and dried under reduced pressure (10-50mum Hg) to obtain Abacavir malonate.
**Yield** : 36 g (88% of theory).
**Chromatographic Purity (by HPLC)** : 99.88%
**Chiral Purity (by HPLC)** :99.95%
**Assay(by HPLC, %w/w)** : 99.9
**Water content (By KF)** : 0.14% w/w
**SOR:** [α]D²⁵ (C=0.5 in Methanol, on anhydrous basis) = -33.5°
**Malonic acid content (on as is basis): 26.70% w/w (Theoretical value-26.67%w/w)**
**¹H-NMR (300MHz) in DMSO-d₆:** α(ppm): 7.64-7.62 (1H, d, N=CH); 7.50 (1H, S, NH); 6.12-6.11 (1H, d, CH=CH); 5.97 (2H, S, NN₂); 5.88-5.86 (1H, dd, CH-CH); 5.40-5.37 (1H, d, CHN); 3.46-3.44 (2H, d, OCH₂); 3.20-3.19 (2H, d, CH₂, Malonate Salt); 3.02 (1H, S, CH-NH); 2.87 (1H, S, CHCH₂0); 2.62-2.58 (1H, m, CH₂, Cyclopentene); 1.61-1.56 (1H, m, CH₂, Cyclopentene); 0.69-0.59 (4H, m, 2CH₂, Cyclopropyl).
Melting range: 148°C -150°C
DSC:
Onset -152.34°C
Peak- 153.34°C

### EXAMPLE-5

### PREPARATION OF CRYSTALLINE FORM OF ABACAVIR MALONATE

Malonic acid (2.36 g, 0.0227 moles) dissolved in 30 ml of methanol was added to a suspension of Abacavir base (5 g, 0.0175 mole) in 30 ml of methanol in a period of -15 min at 25-30°C and stirred to obtain a clear solution. Thereafter, obtained solution was cooled to 0-5°C and stirred for -3 h. Product was filtered, washed with pre-cooled methanol (5ml, 5-10°C) and dried under reduced pressure (∼20mm Hg) to obtain crystalline form of Abacavir malonate.

### EXAMPLE-6

### PREPARATION OF CRYSTALLINE FORM OF ABACAVIR MALONATE

Malonic acid (2.36 g, 0.0227 moles) dissolved in 45 ml of isopropyl alcohol was added to a suspension of Abacavir base (5 g, 0.0175 mole) in 30 ml isopropyl alcohol in a period of∼15min at 25-30°C and further heated to 45-50°C. Thereafter, obtained suspension was cooled to 25-30°C and stirred for -2 h. Product was filtered, washed with isopropyl alcohol (5ml) and dried under reduced pressure (-20mm Hg) to obtain crystalline form of Abacavir malonate.

### EXAMPLE-7

### PREPARATION OF CRYSTALLINE FORM OF ABACAVIR MALONATE

Malonic acid (2.36 g, 0.0227 moles) dissolved in 70 ml of tetrahydofuran was added to a suspension of Abacavir base (5 g, 0.0175 mole) in 30 ml tetrahydofuran in a period of∼15min at 25-30°C and further heated to 45-50°C to obtain a clear solution. Thereafter, obtained solution was cooled to 25-30°C and stirred for -3 h. Product was filtered, washed with tetrahydofuran (10ml) and dried under reduced pressure (-20mm Hg) to obtain crystalline form of Abacavir malonate.

### EXAMPLE-8

### PREPARATION OF CRYSTALLINE FORM OF ABACAVIR MALONATE

Malonic acid (2.36 g, 0.0227 moles) dissolved in 100 ml of acetonitrile was added to a suspension of Abacavir base (5 g, 0.0175 mole) in 50 ml acetonitrile in a period of ∼15min at 25-30°C and further heated to 50-55°C. Thereafter, obtained suspension was cooled to 25-30°C and stirred for -3 h. Product was filtered, washed with acetonitrile (10ml) and dried under reduced pressure (∼20mm Hg) to obtain crystalline form of Abacavir malonate.

### EXAMPLE-9

### PREPARATION OF CRYSTALLINE FORM OF ABACAVIR MALONATE

Abacavir malonate (3.0g, 0.0076moles) suspended in 18 ml of methanol was heated to 45-50°C to obtain a clear solution. Thereafter, 72ml of methyl-tert-butyl ether was added to the obtained clear solution in a period of∼15 min & stirred for ∼1 hr at this temperature to precipitate the product. Thereafter, obtained suspension was cooled to 25-30°C and stirred for ∼2 h. Product was filtered, washed with methanol : methyl-tert-butyl ether (1:4; 10ml) and dried under reduced pressure (∼20mm Hg) to obtain crystalline form of Abacavir malonate.

### EXAMPLE-10

### PREPARATION OF AMORPHOUS FORM OF ABACAVIR MALONATE

Abacavir malonate (5.0g, 0.0128moles), suspended in 50 ml of DM water was stirred 25-30°C to obtain a clear solution and treated with carbon. Thereafter, obtained filtrate was lyophilized at 25-30°C under reduced pressure (50-100 milli torr. of Hg) to obtain Amorphous Abacavir malonate.

### EXAMPLE-11

### PREPARATION OF CRYSTALLINE FORM OF ABACAVIR HEMIOXALATE

Oxalic acid dihydrate (13.88 g, 0.110 mole) was added to suspension of Abacavir base (30 g, 0.105mole) in 450 ml of 20%v/v aqueous ethanol at 25-30°C. Thereafter, the suspension was heated to 70-75°C and stirred for -15 min to obtain a clear solution and then the solution was cooled to 25-30°C to precipitate the product, which was further cooled to 0-5°C and stirred for -2 h. Product was filtered, washed with ethanol (absolute alcohol, 30 ml, 0-5°C) and dried at 40-50°C under reduced pressure (10-50 mm Hg).
**Yield** : 22.5 g (65% of theory).
**Chromatographic Purity (by HPLC)** : 99.84%
**Chiral Purity (by HPLC)** : 99.99%
**Assay (by HPLC, %w/w)** :99.1
**Water content (By KF)** : 0.24% w/w
**SOR**: [α]D²⁵ (C=0.5 in methanol, on anhydrous basis) = -37.9°
**Oxalic acid content (on as is basis):** 13.93% w/w **(Theoretical value=13.60%w/w)**
**¹H-NMR (300MHz) in DMSO-d₆:** α(ppm) 7.79 (1H,S, NH);7.68-7.67 (1H, d, N=CH); 6.13-6.11 (1H, d, CH=CH); 5.88-5.86 (1H, t, CH=CH); 5.40 (1H, S, CH-N); 6.15 & 4.98 (4H, broad peak, NH2, 2 x COOH, OH); 3.46-3.44 (2H, d, OCH₂); 3.03 (1H, S, CH-NH), 2.87 (1H, S, CHCH₂0); 2.63-256 [1H, m, CH₂(cyclopentene)]; 1.61-1.55 [1H, m, CH₂ (cyclopentene)]; 0.71-0.61 (4H, m, 2CH₂, cyclopropyl).
**Melting range :** Decomposed at 213°C
**DSC:**
Onset- 216.42°C
Peak-221.41°C

### EXAMPLE-12

### PREPARATION OF CRYSTALLINE FORM OF ABACAVIR HEMIOXALATE

Abacavir hemioxalate (3 g, 0.0045moles) was suspended in 60 ml of 20%v/v aqueous methanol at 25-30°C. Thereafter, the suspension was heated to 60-65°C and stirred for ∼15 min to obtain a clear solution. Obtained solution was cooled to 25-30°C. Precipitated product was further cooled to 0-5°C and stirred for -2 h. Product was filtered, washed with pre-cooled methanol (6 ml, 0-5°C) and dried at 40-50°C under reduced pressure (20mm Hg) to obtain crystalline form of Abacavir hemioxalate.

### EXAMPLE-13

### PREPARATION OF CRYSTALLINE FORM OF ABACAVIR HEMIOXALATE

Abacavir hemioxalate (2 g, 0.003moles) was suspended in 50 ml of 20%v/v aqueous isopropyl alcohol at 25-30°C. Thereafter, the suspension was heated to 70-78°C and stirred for ∼15 min to obtain a clear solution. Obtained solution was cooled to 25-30°C. Precipitated product was further cooled to 0-5°C and stirred for -2 h. Product was filtered, washed with pre-cooled isopropyl alcohol (4 ml, 0-5°C) and dried at 40-50°C under reduced pressure (20mm Hg) to obtain crystalline form of Abacavir hemioxalate

### EXAMPLE-14

### PREPARATION OF CRYSTALLINE FORM OF ABACAVIR MONOOXALATE (1:1)

Oxalic acid dihydrate (13.88 g, 0.110 moles) was added to a suspension of Abacavir base (15 g, 0.052 moles) in 225 ml of 20% v/v aqueous ethanol at 25-30°C. The contents were heated to 70-75°C and stirred for ∼15 min to obtain a clear solution. Thereafter, obtained clear solution was cooled slowly to 0-5°C and stirred for -2 h at this temperature. Product was filtered, washed with pre-cooled ethanol (30 ml, 0-5°C) and dried at 40-50°C under reduced pressure (10-50 mm Hg).
**Yield :** 13.5 g (68% of theory).
**Chromatographic Purity (by HPLC)** : 99.46%
**Chiral Purity (by HPLC)** : 99.96%
**Assay (by HPLC, %w/w)** : 94.7
**Water content (By KF)** : 0.58% w/w
**SOR**: [α]_{D} ²⁵ (C=0.5 in methanol, on anhydrous basis) : -25.1°
**Oxalic acid content (on as is basis)** : 26.32% w/w **(Theoretical value-23.94%w/w)**
DSC: Triplet:
1) Onset: 68.97°C and Peak: 82.56°C
2) Onset: 145.10°C and Peak: 148.67°C
3) Onset: 159.46°C and Peak: 165.62 °C
**Melting range** : 154°C-157°C
**¹H-NMR (300MHz) in DMSO-d₆** : α(ppm): 7.79 (1H,S, NH);7.71-7.69 (1H, d, N=CH); 6.13-6.11 (1H, d, CH=CH); 5.88-5.86 (1H, t, CH=CH);5.40 (1H, S, CH-N);4.07 (4H, broad peak, NH2, 2 x COOH, OH); 3.45-3.44 (2H, d, OCH₂); 3.00 (1H, S, CH-NH), 2.87 (1H, S, CHCH₂0); 2.63-256 [1H, m, CH₂ (cyclopentene)]; 1.61-1.57 [1H, m, CH₂ (cyclopentene)]; 0.73-0.63 (4H, m, 2CH₂, cyclopropyl).

### EXAMPLE-15

### PREPARATION OF CRYSTALLINE FORM OF ABACAVIR HYDROBROMIDE

48% w/w aqueous hydrobromic acid (3.1 g, 0.0184 mole,) was added to a suspension of Abacavir base (5 g, 0.0175 mole) in 60 ml of ethanol in a period of∼15 min at 25-30°C and stirred for -30 min to obtain a clear solution. Reaction mass was concentrated at 40-50°C under reduced pressure (200-50 mm Hg) to obtain oily mass. Thereafter, tetrahydrofuran (60 ml) was added to concentrated mass and stirred for -30 min at 25-30°C. Precipitated product was cooled to 0-5°C and stirred four∼3 h to complete the precipitation. Product was filtered, washed with pre-cooled tetrahydrofuran (1x 15 ml, 0-5°C) and dried at 40-50°C under reduced pressure (10-50 mm Hg).
**Yield:** 5.25 g (82% theory)
**Chromatographic Purity (by HPLC)** : 99.88%
**Chiral Purity (by HPLC)** : 99.93%
**Assay (by HPLC, %w/w)** : 97.9
**Water content (By KF)** : 0.59% w/w
**SOR**: [α]_{D}²⁵ (C=0.5 in methanol, on anhydrous basis) = -21.2°
**HBr content (on as is basis)**: 22.06% w/w **(Theoretical value=22.07%w/w) Melting range**: Decomposed at 138°C
**DSC:** Triplet:
1) Onset: 48.74°C and Peak: 67.29°C
2) Onset: 143.41°C and Peak: 150.58°C
3) Onset: 153.53°C and Peak: 155.84 °C
**¹H-NMR (300MHz) in DMSO-d₆** : α(ppm): 9.90 (1H, S, NH); 8.01 (1H, S, CH=N); 7.65 (2H, S, NH2); 6.18-6.16 (1H, d, CH=CH); 5.90-5.88 (1H, t, CC=CH); 5.42 (1H, S, N-CH); 3.51-3.45 (2H, t, OCH₂); 2.89 (2H, S, CH-NH and CHCH₂0); 2.69-2.59 [1H, m, CH₂ (cyclopentene)]; 1.67-1.59 [1H, m, CH₂ (cyclopentene)].

### EXAMPLE-16

### PREPARATION OF CRYSTALLINE FORM OF ABACAVIR HYDROBROMIDE MONOHYDRATE

48% w/w aqueous hydrobromic acid (3.1 g, 0.0184 mole) was added to a suspension of Abacavir base (5 g, 0.0175 mole) in 60 ml of ethanol in a period of∼15 min at 25-30°C and stirred for -30 min to obtain a clear solution. Reaction mass was concentrated at 40-50°C under reduced pressure (200-50 mm Hg) to obtain oily mass. Thereafter, tetrahydrofuran (60 ml) was added to concentrated mass and stirred for -30 min at 25-30°C. Precipitated product was cooled to 0-5°C and stirred for -3 h to complete the precipitation. Product was filtered, washed with pre-cooled tetrahydrofuran (1x15 ml, 0-5°C) and dried at 60-65°C under humid atmosphere for -40hrs to obtain crystalline form of Abacavir hydrobromide monohydrate.
**Water content (By KF)** : 4.61 % w/w (Theoretical =4.67%w/w)
DSC: Triplet:
1) Onset: 71.25°C and Peak: 94.18°C
4) Onset: 116.15°C and Peak: 133.03°C
5) Onset: 144.96°C and Peak: 153.82 °C
6) Onset: 221.94°C and Peak: 247.10 °C

### EXAMPLE-17

### PREPARATION OF CRYSTALLINE FORM OF ABACAVIR DIHYDROBROMIDE

48% w/w Aqueous hydrobromic acid solution (6.2 g, 0.0367 moles) was added to a suspension of Abacavir base (5 g, 0.0175 mole) in 60 ml of ethanol in a period of ∼15 min and stirred for -30 min at 25-30°C to obtain a clear solution. Reaction mass was concentrated at 40-50°C under reduced pressure (200-50 mm Hg) to obtain oily mass. Thereafter, tetrahydrofuran (30 ml) was added to concentrated mass and stirred for 30 min at 25-30°C. Precipitated product was cooled to 0-5°C and stirred for -3 h to complete the precipitation. Product was filtered, washed with pre-cooled tetrahydrofuran (1x15 ml, 0-5°C) and dried at 40-50°C under reduced pressure (-20 mm Hg).
**Yield**: 6.5 g (83% theory)
**Chromatographic Purity (by HPLC)** : 99.89%
**Chiral Purity (by HPLC)** : 99.98%
**Assay (by HPLC, % w/w)** : 100.5
**Water content (By KF)** : 0.36% w/w
**SOR**: [a]_{D}²⁵ (C=0.5 in methanol, on anhydrous basis) : -19.6°
**HBr content (on as is basis)** : 35.56% w/w **(Theoretical value =36.16%w/w) Melting range**: Decomposed at 183°C
**DSC**: Triplet:
1) Onset: 158.68°C and Peak: 185.27°C
2) Onset: 196.44°C and Peak: 204.60°C
3) Onset: 229.47°C and Peak: 235.28 °C
**¹H-NMR(300MHz)inDMSO-d₆**: α(ppm): 9.91 (1H, S, NH); 8.01-8.00 (1H, d, CH=N); 7.65 (2H, S, NH2); 6.18-6.16 (1H, d, CH=CH); 5.90-5.88 (1H, t, CC=CH); 5.42 (1H, S, N-CH); 3.47-3.45 (2H, t, OCH₂); 2.89-2.88 (2H, S, CH-NH and CHCH₂0); 2.69-2.59 [1H, m, CH₂ (cyclopentene)];1.67-1.59[1H,m,CH₂ (cyclopentene)]; 0.96-0.80 [4H, dd, 2CH₂ (cyclopropyl)

### EXAMPLE-18

### PREPARATION OF CRYSTALLINE FORM OF ABACAVIR DIHYDROBROMIDE MONOHYDRATE

48% w/w Aqueous hydrobromic acid solution (6.2 g, 0.0367 moles) was added to a suspension of Abacavir base (5 g, 0.0175 mole) in 60 ml of ethanol in a period of -15 min and stirred for -30 min at 25-30°C to obtain a clear solution. Reaction mass was concentrated at 40-50°C under reduced pressure (200-50 mm Hg) to obtain oily mass. Thereafter, tetrahydrofuran (30 ml) was added to concentrated mass and stirred for 30 min at 25-30°C. Precipitated product was cooled to 0-5°C and stirred for -3 h to complete the precipitation. Product was filtered, washed with pre-cooled tetrahydrofuran (1x15 ml, 0-5°C) and dried at 60-65°C under humid atmosphere for -20hrs to obtain crystalline form of Abacavir dihydrobromide monohydrate
**Water content (By KF)** : 4.26% w/w (Theoretical =3.86%w/w)
**DSC**: Triplet:
1) Onset: 117.21 °C and Peak: 142.05°C
3) Onset: 150.68°C and Peak: 151.65°C
4) Onset: 176.81 °C and Peak: 190.10°C
5) Onset: 231.59°C and Peak: 237.10°C

### EXAMPLE-19

### PREPARATION OF CRYSTALLINE FORM OF ABACAVIR MALEATE

Maleic acid (2.45 g, 0.0209 moles) dissolved in 15 ml of ethanol was added to a suspension of Abacavir base (5 g, 0.0175 mole) in 60 ml of ethanol and stirred for 15 min at 25-30°C to obtain a clear solution. Reaction mass was concentrated at 40-50°C under reduced pressure (200-50 mm Hg) to get the oily mass. Thereafter, Isopropyl alcohol (50 ml) was added to concentrated mass and stirred for 40-50°C for -30 min, to precipitate the product. Obtained slurry was cooled to 0-5°C and stirred for ∼2 h to complete the precipitation. Product was filtered, washed with pre-cooled Isopropyl alcohol and dried at 40-50°C under reduced pressure (-20 mm Hg).
**Yield**: 6.6 g (94.3% theory).
**Chromatographic Purity (by HPLC)** : 99.82%
**Chiral Purity (by HPLC)** : 99.96%
**Assay (by HPLC, %w/w)** : 99.0
**Water content (By KF)** : 0.39% w/w
**SOR**: [α]_{D}²⁵ (C=0.5 in methanol, on anhydrous basis) : -22.8°
**Maleic acid content (on as is basis)** : 28.80% w/w **(Theoretical value =28.86%w/w)**
**Melting range**: 134°C-136°C
**DSC**:
On set- 139.29°C
Peak- 141.04°C
**¹H-NMR (300MHz) in DMSO-d₆**: α(ppm): 8.85 [1H, S (broad), NH)]; 7.83 (1H, S, CH=N); 6.65 [2H, S (broad), NH₂); 6.14-6.13 [3H, d, CH=CH and 2CH (maleate salt)]; 5.89-5.87 (1H, t, CH=CH); 5.41 (1H (1H, S, CH-N); 3.46-3.45 (2H, d, OCH₂); 2.89 (2H, S, CH-NH and CHCH₂0); 2.67-2.57 [1H, m, cyclopentene (CH₂)]; 1.65-1.59 [1H, m, cyclopentene (CH₂)]; 0.82-0.70 [4H, dd, 2CH₂ (cyclopropyl)

### EXAMPLE-20

### PREPARATION OF CRYSTALLINE FORM OF ABACAVIR MALEATE

Maleic acid (2.45 g, 0.0209 moles) dissolved in 25 ml of methanol was added to a suspension of Abacavir base (5 g, 0.0175 mole) in 25 ml of methanol and stirred for 15 min at 25-30°C to obtain a clear solution. The contents were heated to 40-50°C and slowly cooled to 0-5°C and stirred for -5 h to complete the precipitation. Product was filtered, washed pre-cooled methanol and dried at 40-50°C under reduced pressure (-20 mm Hg) to obtain crystalline form of Abacavir maleate.

### EXAMPLE-21

### PREPARATION OF CRYSTALLINE FORM OF ABACAVIR MALEATE

Maleic acid (2.45 g, 0.0209 moles) dissolved in 50 ml of ethanol was added to a suspension of Abacavir base (5 g, 0.0175 mole) in 25 ml of ethanol and stirred for 15 min at 25-30°C to obtain a clear solution. The contents were heated to 50-60°C and slowly cooled to 0-5°C and stirred for -3 h to complete the precipitation. Product was filtered, washed pre-cooled ethanol and dried at 40-50°C under reduced pressure (-20 mm Hg) to obtain crystalline form of Abacavir maleate.

### EXAMPLE-22

### PREPARATION OF CRYSTALLINE FORM OF ABACAVIR MALEATE

Maleic acid (2.45 g, 0.0209 moles) dissolved in 50 ml of tetrahydofuran was added to a suspension of Abacavir base (5 g, 0.0175 mole) in 25 ml of tetrahydrofuran and stirred for 15 min at 25-30°C to obtain a clear solution. The contents were heated to 45-50°C and slowly cooled to 25-30°C and stirred for -5 h to complete the precipitation. Product was filtered, washed tetrahydrofuran and dried at 40-50°C under reduced pressure (-20 mm Hg) to obtain crystalline form of Abacavir maleate

### EXAMPLE-23

### PREPARATION OF CRYSTALLINE FORM OF ABACAVIR MALEATE

Maleic acid (2.45 g, 0.0209 moles) dissolved in 75 ml of acetonitrile was added to a suspension of Abacavir base (5 g, 0.0175 mole) in 25 ml of acetonitrile and stirred for 15 min at 25-30°C to obtain a clear solution. The contents were heated to 50-60°C and slowly cooled to 25-30°C and stirred for -3 h to complete the precipitation. Product was filtered, washed acetonitrile and dried at 40-50°C under reduced pressure (-20 mm Hg) to obtain crystalline form of Abacavir maleate.

### EXAMPLE-24

### PREPARATION OF CRYSTALLINE FORM OF ABACAVIR MALEATE

Maleic acid (2.45 g, 0.0209 moles) dissolved in 100 ml of ethyl acetate was added to a suspension of Abacavir base (5 g, 0.0175 mole ) in 50 ml of ethyl acetate. The contents were heated to 70-75°C and stirred for -30 min & filtered to remove undissolved matter. Obtained filtrate was slowly cooled to 25-30°C and stirred for -3 h to complete the precipitation. Product was filtered, washed with ethyl acetate and dried at 40-50°C under reduced pressure (-20 mm Hg) to obtain crystalline form of Abacavir maleate.

### EXAMPLE-25

### PREPARATION OF AMORPHOUS FORM OF ABACAVIR MALEATE

Abacavir maleate (5.0g, 0.0124moles), suspended in 50 ml of DM water was stirred 25-30°C to obtain a clear solution and treated with carbon. Thereafter, obtained filtrate was lyophilized at 25-30°C under reduced pressure (50-100 milli torr of Hg) to obtain Amorphous Abacavir maleate.

### EXAMPLE-26

### PREPARATION OF (1S, 4R)-4-[2-AMINO-(6-CYCLOPROPYLAMINO)-9H-PURIN-9-YL]-2-CYCLOPENTENE OXALATE [ABACAVIR OXALATE (2:1)]

Oxalic acid dihydrate (6.94 g, 0.055 moles) was added to a suspension of Abacavir base (15 g, 0.0525 moles) in 225 ml of 20% v/v aqueous ethanol at 25-30°C and contents were heated to 70-75°C to obtain a clear solution. Obtained solution was cooled to 25-30°C and seeded with Abacavir hemioxalate salt. The contents were cooled to 0-5°C and stirred for -2 h at this temperature to complete the crystallization. Product was filtered, washed with ethanol (15 ml, 0-5°C) and dried at 40-50°C under reduced pressure (10-50 mm Hg).
**Yield**: 9.3 g

### Chromatographic Purity (by HPLC): 99.64%.

### PURIFICATION OF ABACAVIR OXALATE (2:1)

### METHOD-A:

After suspending Abacavir oxalate (2:1) (8 g, 0.024 moles) in a mixture of ethyl acetate (80 ml) and DM water (24 ml), contents were heated to 40-45°C and pH was readjusted to 6.0-6.5 with 10% w/w aqueous sodium hydroxide solution (15 ml) at this temperature. Organic layer was separated and aqueous layer was extracted with ethylacetate. Combined ethylacetate layer was treated with carbon and concentrated at 40-50°C under vacuum to yield foamy solid. Oxalic acid dihydrate (3.2 g, 0.0254 moles) was added to a suspension of Abacavir base in 80 ml of 20% v/v and contents were heated to 70-75°C to obtain a clear solution. The obtained solution was cooled to 25-30°C and seeded with Abacavir hemi-oxalate salt. Contents were cooled to 0-5°C and stirred for -2 h at this temperature to complete the crystallization. Product was filtered, washed with ethanol (16 ml, 0-5°C) and dried at 40-50°C under reduced pressure (10-50 mm Hg).
**Yield**: 5.6 g
**Chromatographic Purity (by HPLC)**: 99.78%.

### METHOD-B:

Abacavir oxalate (2:1) (8 g, 0.024 moles) was suspended in 20% v/v aqueous ethanol (120 ml) and heated to 65-70°C to obtain a clear solution. The obtained solution was slowly cooled to 25-30°C. Precipitated product was further cooled to 0-5°C, filtered, washed with pre-cooled ethanol (16 ml) and dried at 45-50°C under vacuum to a constant weight.
**Yield**: 6.4 g
**Chromatographic Purity (by HPLC)**: 99.81%

### EXAMPLE-27

### PREPARATION OF (1S,4R)-4-[2-AMINO-(6-CYCLOPROPYLAMINO)-9H-PURIN-9-YL]-2-CYCLOPENTENE-1-METHANOL MALONATE (ABACAVIR MALONATE)

Malonic acid (7.1 g, 0.068 moles) dissolved in 75 ml of ethanol was added to a suspension of Abacavir base (15 g, 0.0525 moles) in 150 ml of ethanol at 25-30°C and contents were heated to 60-70°C to obtain a clear solution. Obtained solution was cooled to 40-45°C and seeded with Abacavir malonate and further cooled to 5-10°C and stirred for -2 h at this temperature to complete the crystallization. Product was filtered, washed with ethanol (15 ml, 5-10°C) and dried at 40-50°C under reduced pressure (10-50 mm Hg).
**Yield**: 13.1 g
**Chromatographic Purity (by HPLC): 99.40%**

### PURIFICATIN OF ABACAVIR MALONATE

### METHOD-A:

After suspending Abacavir malonate (10 g, 0.0256 moles) in a mixture of ethylacetate (120 ml) and DM water (30 ml), contents were heated to 40-45°C and pH of biphasic solution was adjusted to 6.0-6.5 with 10% w/w aqueous sodium hydroxide solution (28 ml). Organic layer was separated and aqueous layer was extracted with ethyl acetate by saturating it with sodium chloride. Thereafter, combined organic layer was treated with carbon and concentrated at 40-50°C /200-10 mmHg to yield foamy solid. Malonic acid (3.47g, 0.0333 moles) and ethanol (120 ml) was added to above concentrated mass and contents were further heated to 65-70° C to obtain a clear solution. Obtained solution was slowly cooled to 0-5°C and stirred for - 2 h. Precipitated product was filtered, washed with ethanol (10 ml) and thereafter dried at 40-50°C/∼20mmHg.
**Yield**: 8.5 g
**Chromatographic Purity (by HPLC)**: 99.57%

### METHOD-B:

Abacavir malonate (4 g, 0.0103 moles) was suspended in 5% v/v aqueous ethanol (20 ml) and heated to 65-70°C to get a clear solution. Obtained solution was slowly cooled to 25-30°C. Precipitated product was further cooled to 0-5°C, filtered, washed with pre-cooled ethanol (8 ml) and dried at 45-50°C under vacuum to a constant weight.
**Yield**: 3 g
**Chromatographic Purity (by HPLC)**: 99.70%.

### EXAMPLE-28

### PREPARATION OF (1S,4R)-4-[2-AMINO-(6-CYCLOPROPYLAMINO)-9H-PURIN-9-YL]-2-CYCLOPENTENE-1-METHANOL MALEATE (ABACAVIR MALEATE)

Maleic acid (7.3 g, 0.063 moles, 1.2 mole ratio) dissolved in 45 ml of ethanol was added to a suspension of Abacavir base (15 g, 0.0525 moles) in 180 ml of ethanol at 25-30°C and stirred to obtain a clear solution. After precipitation contents were further cooled to 5-10°C and stirred for -2 h at this temperature to complete the crystallization. Product was filtered, washed with pre-cooled ethanol and dried at 40-50°C under reduced pressure (10-50 mm Hg).
**Yield**: 9.3 g
**Chromatographic Purity (by HPLC)**: 99.24%.

### PURIFICATIN OF ABACAVIR MALEATE

### METHOD-A:

After suspending Abacavir maleate (8 g, 0.0199 moles) in a mixture of ethyl acetate (80 ml) and DM water (24 ml), and heated to 40-45°C. The pH of biphasic solution was adjusted to 6.0-6.5 with 10% w/w aqueous sodium hydroxide solution (16 ml). Organic layer was separated and aqueous layer was extracted with ethyl acetate by saturating it with sodium chloride. Thereafter, combined organic layer was treated with carbon and concentrated at 40-50°C/200-10 mmHg to yield foamy solid. Maleic acid (2.77 g, 0.0239 moles) and ethanol (120 ml) were added to above concentrated mass and contents were slowly cooled to 0-5°C and stirred for - 2 h. Precipitated product was filtered, washed with ethanol (16 ml) and thereafter dried at 40-50°C/-20mmHg.
**Yield**: 5.9 g
**Chromatographic Purity (by HPLC)**: 99.46%.

### METHOD-B:

Abacavir maleate (7 g, 0.0174 moles) was suspended in 5% v/v aqueous ethanol (112 ml) and contents were heated to 25-30°C to obtain a clear solution. The obtained solution was slowly cooled to 0-5°C and stirred for -4 h to complete the precipitation. Precipitated product was filtered, washed with pre-cooled ethanol (8 ml) and dried at 45-50°C under vacuum to a constant weight.
**Yield**: 3.5 g
**Chromatographic Purity (by HPLC)**: 99.68%

### EXAMPLE-29

### PREPARATION OF (1S, 4R)-4-[2-AMINO-(6-CYCLOPROPYLAMINO)-9H-PURIN-9-YL]-2-CYCLOPENTENE OXALATE [ABACAVIR OXALATE (2:1)]

Oxalic acid dihydrate (26.43 g, 0.2098 moles, 0.5 mole ratio) was added to a suspension of Abacavir base (120 g, 0.4195 moles) in 1800 ml of 20% v/v aqueous ethanol at 25-30°C and contents were heated to 70-75°C to obtain a clear solution & thereafter treated with carbon enoanticromos. The obtained solution was cooled to 20-30°C and stirred for -2 hrs, which was further cooled to 0-5°C and stirred for ∼2 h at this temperature to complete the crystallization. Product was filtered, washed with ethanol (120 ml, 10-15°C) and dried at 40-50°C under reduced pressure (10-50 mm Hg).
**Yield**: 94.7 g
**Chromatographic Purity (by HPLC)**: 99.29%

### PURIFICATION OF ABACAVIR OXALATE (2:1)

Abacavir oxalate (2:1) (40 g, 0.1208 moles) was suspended in 20% v/v aqueous ethanol (600 ml) and heated the contents to 65-70°C to obtain a clear solution. The obtained solution was cooled slowly to 10-15°C and stirred for -2 h to complete the precipitation. Filtered the product, washed with ethanol (40 ml) and dried at 40-50°C under vacuum.
**Yield**: 32.8 g
**Chromatographic Purity (by HPLC)**: 99.70%

### EXAMPLE-30

### PREPARATION OF (1S,4R)-4-[2-AMINO-(6-CYCLOPROPYLAMINO)-9H-PURIN-9-YL]-2-CYCLOPENTENE-1-METHANOL MALONATE) (ABACAVIR MALONATE)

Abacavir oxalate (2:1) (20 g, 0.0604 moles) was suspended in a mixture of ethyl acetate (200 ml) and DM water (60 ml) and heated to 40-45°C. The pH of the solution was adjusted to 6.0-6.5 with 10% w/w aqueous sodium hydroxide solution (48 ml) at this temperature. Organic layer was separated and aqueous layer was extracted with ethyl acetate. Combined ethylacetate layer was treated with carbon and concentrated at 40-50°C under vacuum to yield foamy solid. Malonic aid (6.6 g, 0.0634 moles, 1.05 mole ratios) and ethanol (300 ml) were added and contents were heated to 65-70°C to obtain a clear solution. Obtained solution was slowly cooled to 15-20°C and stirred for -2 h at this temperature to complete the crystallization. Product was filtered, washed with ethanol (20 ml) and dried at 40-50°C under reduced pressure (10-50 mm Hg).
**Yield**: 18.4 g
**Chromatographic Purity (by HPLC)**: 99.88%

### EXAMPLE-31

### PREPARATION OF (1S,4R)-4-[2-AMINO-(6-CYCLOPROPYLAMINO)-9H-PURTN-9-YL] 2-CYCLOPENTENE-1-METHANOL MALEATE (ABACAVIR MALEATE)

After suspending Abacavir oxalate (2:1) (25 g, 0.0755 moles) in a mixture of ethylacetate (250 ml) and DM water (75 ml), contents were heated to 40-45°C and pH was adjusted to 6.0-6.5 with 10% w/w aqueous sodium hydroxide solution (60 ml) at this temperature. The organic layer was separated and aqueous layer was extracted with ethylacetate. Thereafter, combined ethylacetate layer was treated with carbon and then concentrated at 40-50°C under vacuum to yield foamy solid. Maleic acid (9.20 g, 0.0793 moles, 1.05 mole ratio) and ethanol (300 ml)) were added and contents were stirred at 25-30°C to obtain a clear solution. Obtained solution was slowly cooled to 10-12°C and stirred for -2 h at this temperature to complete the crystallization. Product was filtered, washed with ethanol (25 ml) and dried at 40-50°C under reduced pressure (10-50 mm Hg).
**Yield**: 22.5 g
**Chromatographic Purity (by HPLC)**: 99.78%

### EXAMPLE-32

### PREPARATION OF (1S, 4R)-4-[2-AMINO-(6-CYCLOPROPYLAMINO)-9H-PURIN-9-YL]-2-CYCLOPENTENE OXALATE [ABACAVIR OXALATE (2:1)]

Oxalic acid dihydrate (6.6 g, 0.0524 moles, 0.5 mole ratio) was added to a suspension of Abacavir base (30 g, 0.1049 moles) in 540 ml of 20% v/v aqueous ethanol at 25-30°C and contents were heated to 70-75°C to obtain a clear solution and treated with carbon enoanticromos . The obtained solution was cooled to 20-30°C and stirred for -2 hrs and further cooled to 0-5°C and stirred for -2 h at this temperature to complete the crystallization. Product was filtered, washed with ethanol (120 ml, 10-15°C) and dried at 40-50°C under reduced pressure (10-50 mm Hg).
**Yield**: 25.82 g
**Chromatographic Purity (by HPLC)**: 99.84%

### EXAMPLE-33

### PREPARATION OF (1S,4R)-4-[2-AMINO-(6-CYCLOPROPYLAMINO)-9H-PURIN-9-YL]-2-CYCLOPENTENE-1-METHANOL MALONATE (ABACAVIR MALONATE)

Malonic acid (5.72 g, 0.055 moles, 1.05 mole ratios) dissolved in 75 ml of ethanol was added to a suspension of Abacavir base (15 g, 0.0525 moles) in 150 ml of ethanol at 25-30°C and contents were heated to 60-70°C to obtain a clear solution. The obtained solution was cooled to 40-45°C and seeded with Abacavir malonate and further cooled to 15-20°C and stirred for -2 h at this temperature to complete the crystallization. Product was filtered, washed with ethanol (15 ml, 15-20°C) and dried at 40-50°C under reduced pressure (10-50 mm Hg).
**Yield**: 16.3 g
**Chromatographic Purity (by HPLC)**: 99.78%

### EXAMPLE-34

### PREPARATION OF (1S,4R)-4-[2-AMINO-(6-CYCLOPROPYLAMINO)-9H-PURIN-9-YL]-2-CYCLOPENTENE-1-METHANOL MALEATE (ABACAVIR MALEATE)

Maleic acid (6.39 g, 0.055 moles, 1.05 mole ratio) dissolved in 45 ml of ethanol was added to a suspension of Abacavir base (15 g, 0.0525 moles) in 180 ml of ethanol at 25-30°C and stirred to obtain a clear solution. After precipitation contents were further cooled to 15-20°C and stirred for -2 h at this temperature to complete the crystallization. Product was filtered, washed with pre-cooled ethanol and dried at 40-50°C under reduced pressure (10-50 mm Hg).
**Yield**: 13.25 g
**Chromatographic Purity (by HPLC)**: 99.83%

### EXAMPLE-35

### PREPARATION OF ABACAVIR MALEATE TABLETS 300MG

| **S. No.** | **Ingredients** | **mg/tab** |
|---|---|---|
| 1 | Abacavir Maleate (equivalent to Abacavir 300mg) | 422.26 |
| 2 | Corn starch | 120.00 |
| 3 | Microcrystalline Cellulose | 323.39 |
| 4 | Sodium Starch Glycolate | 39.00 |
| 5 | Colloidal silicon dioxide | 11.70 |
| 6 | Hypromellose | 5.85 |
| 7 | Magnesium Stearate | 7.80 |
| 8 | Ethanol | q.s. |
| | **Core Tablet weight** | **930.00** |
| 9 | Opadry | 20.00 |
| | **Coated Tablet weight** | **950.000** |

Manufacturing process:
a) Abacavir maleate, corn starch, a portion of microcrystalline cellulose, a portion of sodium starch glycolate and a portion of colloidal silicon dioxide are sifted through suitable mesh and then blended,
b) hypromellose is dissolved in ethanol and the blend of step (a) is granulated with the hypromellose solution,
c) the granules are dried, milled and sifted,
d) dried granules of step (c) are blended with sifted remaining portions of sodium starch glycolate, colloidal silicon dioxide and microcrystalline cellulose,
e) the blend of step (d) is lubricated with sifted magnesium stearate and compressed to form tablet dosage form and
f) the compressed tablets of step (e) are coated with opadry coating.

### EXAMPLE-36

### PREPARATION OF ABACAVIR OXALATE TABLETS 300MG

| **S. No.** | **Ingredients** | **mg/tab** |
|---|---|---|
| 1 | Abacavir Oxalate (equivalent to Abacavir 300mg) | 347.72 |
| 2 | Colloidal silicon dioxide | 4.50 |
| 3 | Magnesium Stearate | 5.00 |
| 4 | Microcrystalline Cellulose | 395.78 |
| 5 | Sodium Starch Glycolate | 24.00 |
| 6 | Magnesium Stearate | 3.00 |
| | **Core Tablet weight** | **780.00** |
| 7 | Opadry | 20.00 |
| | **Coated Tablet weight** | **800.000** |

Manufacturing process:
a) Abacavir oxalate, colloidal silicon dioxide and magnesium stearate are sifted and blended,
b) microcrystalline cellulose and sodium starch glycolate are sifted and blended,
c) blends of step (a) and (b) are blended together,
d) the blend of step (c) is lubricated with sifted magnesium stearate and compressed to form tablet dosage form and
e) the tablets of step (d) are coated with opadry coating.

### EXAMPLE-37

### PREPARATION OF ABACAVIR MALONATE TABLETS 300MG

| **S. No.** | **Ingredients** | **mg/tab** |
|---|---|---|
| 1 | Abacavir Malonate (equivalent to Abacavir 300mg) | 414.48 |
| 2 | Corn starch | 120.00 |
| 3 | Microcrystalline Cellulose | 184.82 |
| 4 | Sodium Starch Glycolate | 39.00 |
| 5 | Hypromellose | 10.00 |
| 6 | Colloidal silicon dioxide | 3.90 |
| 7 | Magnesium Stearate | 7.80 |
| 8 | Purified water | q.s. |
| | **Core Tablet weight** | **780.00** |
| 9 | Opadry | 20.00 |
| | **Coated Tablet weight** | **800.000** |

Manufacturing process:
a) Abacavir malonate, corn starch, microcrystalline cellulose and a portion of sodium starch glycolate are sifted through suitable mesh and then blended,
b) hypromellose is dissolved in purified water and the blend of step (a) is granulated with the hypromellose solution,
c) the granules are dried, milled and sifted,
d) dried granules of step (c) are blended with sifted remaining portion of sodium starch glycolate and colloidal silicon dioxide,
e) the blend of step (d) is lubricated with sifted magnesium stearate and compressed to form tablet dosage form and
f) the tablets of step (e) are coated with opadry coating.

The tablet formulations of Examples 34 to 36 and marketed formulation Ziagen® are subjected to dissolution in 900 ml of 0.1N HC1 at 75rpm using USP paddle apparatus.

**Table: Comparison of Dissolution profile for tablets as per Examples 34-36 with marketed formulation Ziagen®**

| **Time points** **(min.)** | **% drug release in 0.1N HCl/900ml/ 75rpm/ paddle** | | | |
|---|---|---|---|---|
| | **Abacavir Maleate tablets** **(Example-35)** | **Abacavir Oxalate tablets** **(Example-36)** | **Abacavir Malonate tablets** **(Example-37)** | **Abacavir sulfate tablets 300mg (Ziagen®)** **(Batch No. 3ZP2597)** |
| 5 | 59 | 100 | 83 | 101 |
| 10 | 97 | 101 | 100 | 101 |
| 15 | 100 | 100 | 102 | 101 |
| 30 | 101 | 100 | 101 | 101 |
| 45 | 100 | - | - | 101 |

## Claims

1. Salt of (1S,4R)-cis-4-[2-amino-6-(cyclopropylamino)-9H-purin-9-yl]-2-cyclopentene-1-methanol of Formula I 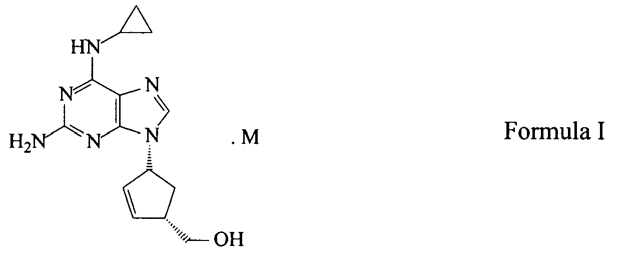 or solvates or hydrates thereof,
wherein M represents hemimalonate, malonate, hemioxalate, oxalate, hydrobromide, dihydrobromide, hemimaleate, or maleate.

2. The salt of (1S,4R)-cis-4-[2-amino-6-(cyclopropylamino)-9H-pwin-9-yl]-2-cyclopentene-1-methanol or solvates or hydrates thereof according to claim 1, in crystalline or amorphous form.

3. Amorphous Abacavir malonate and amorphous Abacavir maleate according to claim 1.

4. The process to prepare amorphous Abacavir malonate or amorphous Abacavir maleate according to claim 3, comprising:
i) treating Abacavir in a solvent with malonic acid or with maleic acid, respectively;
ii) removing the solvent; and
iii) isolating the Abacavir malonate salt or Abacavir maleate salt, respectively, wherein the Abacavir utilized in step i) is in form of the free base or in form of a salt other than the Abacavir salt obtained in step iii).

5. The process according to claim 4, wherein the removal of solvent is carried out using distillation, spray-drying, freeze-drying or lyophilization.

6. Crystalline Abacavir malonate according to claim 1 or 2 having Powder X-ray diffraction °2θ values at 9.47, 13.2, 14.2, 15.6, 17.5, 18.5, 21.6, 22.5, 23.1, 25.2, 26.1, 27.0, 28.6 ±0.2 and a DSC endothermic peak at 153.3°C.

7. Crystalline Abacavir hemioxalate according to claim 1 or 2 having Powder X-ray diffraction °2θ values at 9.4, 12.1, 12.5, 16.9, 17.1, 18.1, 18.6, 18.9, 19.9, 21.3, 21.8, 22.5, 23.4, 23.8, 28.4, 33.9, 36.1 ±0.2 and DSC endothermic peaks at 164.3°C and 221.4°C.

8. Crystalline Abacavir oxalate according to claim 1 or 2 having Powder X-ray diffraction °2θ values at 6.4, 9.1, 17.1, 23.4, 27.5 ±0.2 and DSC endothermic peaks at 82.5°C, 148.6°C and 165.6°C.

9. Crystalline Abacavir maleate according to claim 1 or 2 having Powder X-ray diffraction °2θ values at 10.2, 12.0, 12.6, 14.3, 15.8, 16.4, 17.6, 18.4, 19.2, 20.5, 21.2, 22.5, 23.0, 24.2, 25.7, 26.5, 27.4, 27.7, 28.5 ±0.2 and DSC endothermic peak at 141.0°C.

10. Crystalline Abacavir hydrobromide according to claim 1 or 2 having Powder X-ray diffraction °2θ values at 6.9, 11.8, 15.7, 16.3, 18.4, 18.6, 19.1, 21.0, 21.6, 22.4, 22.8, 23.1, 23.5, 23.9, 25.0, 25.6, 26.5, 27.1 ±0.2 and DSC endothermic peaks at 67.2°C, 150.5°C and exothermic peak at 155.8°C.

11. Crystalline Abacavir dihydrobromide according to claim 1 or 2 having Powder X-ray diffraction °2θ values at 7.6, 15.1, 18.0, 18.3, 20.8, 21.2, 25.1, 26.3, 29.3 ±0.2 and DSC endothermic peak at 185.2°C and exothermic peaks at 204.6°C and 235.2°C.

12. Crystalline Abacavir hydrobromide monohydrate according to claim 1 or 2 having Powder X-ray diffraction °2θ values at 7.5, 9.2, 11.8, 13.4, 14.3, 16.3, 17.7, 18.6, 19.1, 19.6, 21.3, 21.6, 22.3, 23.0, 23.7, 24.1, 25.1, 25.8, 26.0, 26.5, 26.8, 28.0, 29.9, 30.5, 31.1, 31.6, 32.4, 36.5, 36.9 ±0.2 and DSC endothermic peaks at 94.1°C, 133.0°C and exothermic peak at 153.8°C, 247.1 °C.

13. Crystalline Abacavir dihydrobromide monohydrate according to claim 1 or 2 having Powder X-ray diffraction °2θ values at 7.1, 14.2, 18.8, 21.4, 21.7, 23.3, 24.8, 25.7, 26.8, 27.9, 31.3 ±0.2 and DSC endothermic peaks at 142.0°C and exothermic peak at 151.6°C, 190.1°C, 237.1°C.

14. A process to prepare crystalline form of an Abacavir salt as well as solvates and hydrates thereof according to claim 1 comprising:
i) treating Abacavir in a solvent with the corresponding acid;
ii) isolating the Abacavir salt,
wherein the Abacavir utilized in step i) is in form of the free base or in form of a Abacavir salt other than the Abacavir salt obtained in step ii).

15. A method of treating retroviral infections and/or hepatitis B viral infections comprising administering a compound selected from the group of Abacavir salts as well as solvates and hydrates thereof as claimed by claim 1.

16. A pharmaceutical formulation comprising as active ingredient, a compound selected from the group of Abacavir salts as well as solvates and hydrates thereof as claimed by claim 1 in combination with a pharmaceutically acceptable carrier.

17. A pharmaceutical formulation as claimed in claim 16, wherein pharmaceutically acceptable carrier is selected from the group consisting of binders, glidants, lubricants, diluents, disintegrants, solvents and surface active agents.

18. A pharmaceutical formulation according to claim 16 or 17 adapted for oral, topical or parenteral administration.

19. A pharmaceutical formulation according to claim 17, 18 or 19, in the form of a tablet, capsule, powder, granules or liquid formulation
